(19) Europäisches Patentamt
European Patent Office
Office européen des brevets

(11) Publication number: 0 438 311 A2

# (12) EUROPEAN PATENT APPLICATION

(21) Application number: 91300363.8

(22) Date of filing: 17.01.91

(51) Int. Cl.$^5$: C07K 5/02, A61K 37/64

(30) Priority: 19.01.90 US 467476

(43) Date of publication of application: 24.07.91 Bulletin 91/30

(84) Designated Contracting States: CH DE FR GB IT LI NL

(71) Applicant: MERCK & CO. INC.
126, East Lincoln Avenue P.O. Box 2000
Rahway New Jersey 07065-0900 (US)

(72) Inventor: Williams, Peter D.
260 Shady Nook Road
Harleysville, PA 19438 (US)

(74) Representative: Thompson, John Dr. et al
Merck & Co., Inc. European Patent
Department Terlings Park Eastwick Road
Harlow, Essex CM20 2QR (GB)

## (54) Di- and tripeptide renin inhibitors.

(57) Di- and tripeptide enzyme inhibitors of the formula:

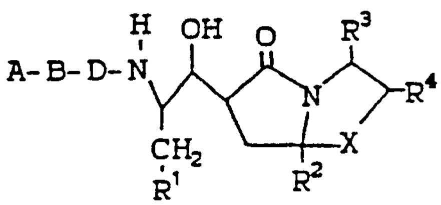

and analogs thereof, which inhibit renin and are useful for treating various forms of renin-associated hypertension, hyperaldosteronism and congestive heart failure; compositions containing these renin-inhibitory peptides, optionally with other antihypertensive agents; and methods of treating hypertension, hyperaldosteronism or congestive heart failure or of establishing renin as a causative factor in these problems which employ these novel peptides.

In addition, these renin inhibitors are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines.

EP 0 438 311 A2

# DI- AND TRIPEPTIDE RENIN INHIBITORS

The present invention is concerned with novel di- and tripeptides, which inhibit the angiotensinogen-cleaving action of the proteolytic enzyme, renin, with pharmaceutical compositions containing the novel peptides of the present invention as active ingredients, with methods of treating renin-associated hypertension, hyperaldosteronism, and congestive heart failure, with diagnostic methods which utilize the novel peptides of the present invention, and with methods of preparing the novel peptides of the present invention.

The present invention is further concerned with compounds which inhibit the protease encoded by human immunodeficiency virus (HIV) or pharmaceutically acceptable salts thereof and are of value in the prevention of infection by HIV, the treatment of infection by HIV, and the treatment of the resulting acquired immune deficiency syndrome (AIDS).

It also relates to pharmaceutical compositions containing the compounds and to a method of use of the present compounds and other agents for the treatment of AIDS.

## BACKGROUND OF THE INVENTION

Renin is an aspartic proteinase (molecular eight about 40,000) produced and secreted by the juxtaglomerular cells of the kidney. Renin has a high specificity for and cleaves the naturally- occurring plasma glycoprotein, angiotensinogen, at only the 10, 11 peptide bond, i.e., between the 10th (Leu) and 11th (Leu) amino acid residues in the equine substrate, as described by Skeggs et al, J. Exper. Med. 1957, 106, 439, or between Leu 10 and Val 11 in the human renin substrate, as elucidated by Tewksbury et al., Circulation 59, 60, Supp. II : 132, Oct. 1979.

This cleavage of its protein substrate, angiotensinogen, by the endopeptidase, renin, splits off the decapeptide, angiotensin I, which itself is thought to be hemodynamically-inactive, but which is converted in the lungs, kidneys or other tissue by the peptidase, angiotensin-converting enzyme (ACE), to the potent vasopressor octapeptide, angiotensin II. Thus released, the hormone, angiotensin II, then causes constriction of the arterioles and is also believed to stimulate release of the sodium-retaining hormone, aldosterone, from the adrenal cortex, thereby causing a rise in extracellular fluid volume. Accordingly, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the catalytic and rate-limiting enzymatic step that ultimately regulates angiotensin II production, the action of renin on its substrate, have also been sought as effective investigative tools, as well as therapeutic agents in the treatment of hypertension and congestive heart failure.

Renin antibody, pepstatin, phospholipids, and substrate analogs, including tetrapeptides and octa-to tridecapeptides, with inhibition constants ($K_i$) in the $10^{-3}$ to $10^{-6}$M region, have been studied.

Umezawa et al., in J. Antibiot. (Tokyo) 23 : 259-262, 1970, reported the isolation of a peptide, pepstatin, from actinomyces that was an inhibitor of aspartyl proteases such as pepsin, cathepsin D, and renin. Gross et al., Science 175 :656, 1972, reported that pepstatin reduces blood pressure in vivo after the injection of hog renin into nephrectomized rats. However, pepstatin has not found very wide application as an experimental agent because of its limited solubility and its inhibition of a variety of other acid proteases in addition to renin.

Many efforts have been made to prepare a specific renin inhibitor based on pig renin substrate analogy, since such analogy has been shown to correlate well with and predict human renin inhibitor activity. The octapeptide amino acid sequence extending from histidine-6 through tyrosine-13

```
  6   7   8   9   10  11  12  13
(-His-Pro-Phe-His-Leu-Leu-Val-Tyr-)
```

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate have been also synthesized, e.g., Burton et al., Biochemistry 14 : 3892-3898, 1975, and Poulsen et al., Biochemistry 12 : 3877-3882, 1973, but a lack of solubility and weak binding (large inhibitory constant) generally resulted.

Modifications to increase solubility soon established that the inhibitory properties of the peptides are markedly dependent on the hydrophobicity of various amino acid residues. These modifications also established that increasing solubility by replacing lipophilic amino acids with hydrophilic isosteric residues can become counter-productive. Other approaches to increasing solubility have also had limited success.

Modifications designed to increase binding to renin have also been made, but here too, with mixed results. A series of inhibitors of renin have been disclosed which contain the unnatural amino acid, statine or its analogs : see, e.g., Veber et al, U.S. Patents 4,384,994 and 4,478,826 ; Evans et al, U.S. Patent 4,397,786 ; Boger et al, Nature, 1983, 303, 81-84 and U.S. Patents 4,470,971 ; 4,485,099 ; 4,663,310 and 4,668,770 ; Matsueda et al, EP-A 128 762, 152 255 ; Morisawa et al., EP-A 186 977 ; Riniker et al, EP-A 111 266 ; Bindra et al, EP-A 155 809 ; Stein et al, Fed. Proc. 1986, 45, 869 ; and Hölzemann et al, German Offenlegungsschrift DE 3438545. Attempting to explain the effect of statine, Powers et al., in Acid Proteases, Structure. Function and Biology, Plenum Press, 1977, 141-157, observed that in pepstatin, statine occupies the space of the two amino acids on either side of the cleavage site of a pepsin substrate and Tang et. al., in Trends in Biochem. Sci., 1 :205-208 (1976) and J. Biol. Chem., 251 :7088-94, 1976, suggested that the statine residue of pepstatin resembles the transition state for pepsin hydrolysis of peptide bonds.

Renin inhibitors containing othr peptide bond isosteres, including a reduced carbonyl isostere, have been disclosed by M. Szelke et al, in work described in published European Patent Applications 45 665 and 104 041; in U.S. Patent 4,424,207, and in PCT Int. Appl. WO 84/03044 ; in Nature, 299, 555 (1982) ; Hypertension, 4, Supp. 2, 59, 1981 ; and British Patent 1,587,809. In Peptides, Structure and Function : Proceedings of the Eighth American Peptide Symposium, ed. V. J. Hruby and D. H. Rich, p. 579, Pierce Chemical Co., Rockford, IL., 1983, Szelke et al also showed isosteric substitutions at the Leu-Leu site of cleavage, resulting in compounds with excellent potency. These and other peptide bond isosteres have also been disclosed in Buhlmayer et al in EP-A 144 290 and 184 550 ; Hester et al, EP-A 173 481 ; Raddatz, EP-A 161 588 ; Dann et al, Biochem. Biophys. Res. Commun. 1986, 134, 71-77 ; Fuhrer et al, EP-A 143 746 ; Kamijo et al, EP-A 181 110 ; Thaisrivongs et al, J. Med. Chem., 1985, 28, 1553-1555 ; Ryono et al., EP-A 181 071 ; and Evans et al, U.S. Patent 4,609,641.

Other modifications which have been tried include preparing renin inhibitors with non-peptide C-termini, such as disclosed in European Published Applications 172 346 and 172 347 ; Evans et al, J. Med. Chem., 1985, 28, 1755-1756 ; and Bock et al, Peptides, Structure and Function : Proceedings of the Ninth American Peptide Symposium, ed. C. M. Deber et al, pp.751-754, Pierce Chemical Co., Rockford, IL, 1985. Kokubu et al, in Hypertension, 1985, 7, Suppl. I, p. 8-10 and Matsueda et al, in Chemistry Letters, 1985, 1041-1044 and in European Published Applications 128 762 and 152 255 disclosed peptide aldehyde renin inhibitors, and Hanson et al in Biochem. Biophys. Res. Commun. 1985, 132, 155-161, reported peptide glycol inhibitors.

These various renin inhibitors all generally comprise peptide-based inhibitors in which a sequence of the type : ...A-B-D-E-F-G-J-K-L... , where G is a peptide bond mimic and A,B,D,E,F,J,K, and L may individually be absent or may represent naturally- occuring or modified amino acids. Typical sequences of this type include :

```
     7   8   9   10  11  12
...BOC-Pro-Phe-His-Sta-Leu-Phe... or,
```

```
    8   9   10  11
...BOC-Phe-His-Sta-Leu... , where the N-terminus
```

typically comprises an amino acid protecting group such as BOC or CBZ, and the N-terminal amino acids are Pro-Phe-His or Phe-His.

Lower molecular weight renin-inhibitory di-or tripeptides comprising acyclic 2-substituted-4-amino-5-cyclohexyl-3-hydroxy-pentanoic acid (ACHPA) have been disclosed in U.S. Patent Application 45,941, filed May 4, 1987, and other lower molecular weight peptides have been disclosed in Sham, EP 184 855, Bindra et al, EP 155 809, and Matsueda et al, EP 152 255. U.S. Patent Applications 108,343 and 108,344, filed October 14, 1987, and Bindra et al, EP 229,667, also disclosed shortened peptides with modified C-terminii.

It was an object of this invention to prepare lower molecular weight peptides which have enhanced biological potency in inhibiting the renin enzyme. It was also an object to prepare shortened peptide sequences which incorporate at the C-terminus a metabolically-stabilizing, conformationally-constrained tripeptide mimic to replace the 10-, 11- and 12-position amino acids in the analogous natural substrate. It was a further object to include strategically-located substituents at the C-termini of a shortened peptide which confer increased potency while constructively altering the physical properties of these peptides. It was an additional object of this invention to prepare peptides which have greater oral bioavailability and increased duration of action. It

was still a further object of this invention to prepare novel peptides which are more useful antihypertensive agents, and compounds useful in treating hyperaldosteronism and congestive heart failure.

It was a further object of this invention to prepare compounds which have utility in the inhibition of HIV protease, the prevention of infection by HIV, the treatment of infection by HIV, and the treatment of AIDS (acquired immune deficiency syndrome), ARC (AIDS related complex) and diseases of similar etiology.

## DETAILED DESCRIPTION OF THE INVENTION

The present invention is directed to renin-inhibitory di- and tripeptides of the structure :

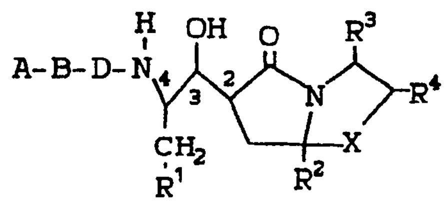

(I)

wherein :

A is hydrogen ;

$C_1$-$C_6$-alkyl ;

aryl, where aryl is unsubstituted or mono-, di- or trisubstituted phenyl, wherein the substituent(s) is/are independently selected from the group consisting of $C_1$-$C_4$-alkyl, phenyl-$C_1$-$C_4$-alkyl, amino, mono- or di-$C_1$-$C_4$-alkylamino, amino-$C_1$-$C_4$-alkyl, hydroxy-$C_1$-$C_4$-alkyl, mono- or di-$C_1$-$C_4$- alkylamino-$C_1$-$C_4$-alkyl, guanidyl, guanidyl-$C_1$-$C_4$-alkyl, hydroxyl,

$C_1$-$C_4$-alkoxy, trifluoromethyl, halo, CHO, $-CO_2H$, $-CONH_2$, -CO-N O, -CONH-$C_1$-$C_4$-alkyl, -CON($C_1$-$C_4$-alkyl$)_2$, -CO-$C_1$-$C_4$-alkyl,

$-(CH_2)_m-{}^+N(R^5)_2R^6\ A^-$, where $R^5$ is $C_1$-$C_4$-alkyl ; $-(CH_2)_p-$, wherein p is 4-to-6 ; or $-(CH_2)_2-O-(CH_2)_2-$ ; $R^6$ is $C_1$-$C_4$-alkyl, hydroxy-$C_1$-$C_4$-alkyl, carboxy-$C_1$-$C_4$-alkyl, carbo-$C_1$-$C_4$- alkoxy-$C_1$-$C_4$-alkyl, or-$CH_2$-phenyl ;

$A^-$ is a counterion selected from the group consisting of single negatively-charged ions, such as chloride, bromide, perchlorate, benzoate, benzene sulfonate, tartrate, maleate, hemitartate, and acetate ;

and m is 0-to-3; $-CO_2$-$C_1$-$C_4$-alkyl, $-CO_2$-$C_1$-$C_4$-alkoxy-$C_2$-$C_4$-alkyl, $-(CH_2)_m-{}^+N$ $A^-$, where $A^-$ and m are as defined above, and

$-NR^7R^8$, where $R^7$ and $R^8$ are independently hydrogen or unsubstituted or monosubstituted $C_1$-$C_4$-alkyl, wherein the substituent is amino, mono- or di-$C_2$-$C_4$-alkylamino or $-{}^+N(R^5)_2R^6\ A^-$, where $R^5$, $R^6$ and $A^-$ are as defined above ;

Het, where Het is an unsubstituted or mono-or disubstituted 5-to-7-membered monocyclic or 7-to-10-membered bicyclic heterocyclic ring, wherein the one or two heteroatoms are independently selected from the group consisting of N, O, S, NO, SO, $SO_2$ and quaternized N, and the substituent(s), when attached to carbon atom(s) in the heterocyclic ring, is/are independently selected from the group consisting of hydroxyl, thiol, $C_1$-$C_6$-alkyl, $CF_3$, aryl- $C_1$-$C_4$-alkoxy, halo, aryl or $C_1$-$C_4$-alkyl, where aryl is as defined above, amino, mono- or di-$C_1$-$C_4$-alkylamino, amino-$C_1$-$C_4$-alkyl, hydroxy-$C_1$-$C_4$-alkyl, mono- or di-$C_1$-$C_4$-alkyl-amino-$C_1$-$C_4$-alkyl, guanidyl, guanidyl-$C_1$-$C_4$-alkyl, CHO, $CO_2H$, $CO_2$-$C_1$-$C_4$-alkyl, $CONH_2$,

$CONH-C_1-C_4$-alkyl, $CON(C_1-C_4$-alkyl$)_2$, $-CO-$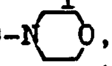, $NR^7R^8$, $-(CH_2)_m-^{+}$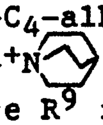 $A^-$ and $-(CH_2)_m-^{+}N(R^5)_2R^9$ $A^-$, where $R^9$ is

$C_1$-$C_4$-alkyl, hydroxy-$C_1$-$C_4$-alkyl, carboxy-$C_1$-$C_4$-alkyl, carbo-$C_1$-$C_4$-alkoxy-$C_1$-$C_4$-alkyl or -$CH_2$-phenyl, wherein $R^7$, $R^8$, $A^-$, m and $R^5$ are as defined above, or, when attached to $sp^3$ hybridized heteroatom nitrogen(s) in the heterocycle ring, is/are independently selected from the group consisting of hydrogen ; unsubstituted or mono-substituted $C_1$-$C_7$-alkyl, where the substituent is independently selected from the group consisting of hydroxyl, amino, mono- or di-$C_1$-$C_4$-alkyl-amino, -$CO_2$H, -$CONH_2$, -CO-NH-$C_1$-$C_4$-alkyl, -CON($C_1$-$C_4$-

alkyl$)_2$, -CO-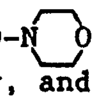, -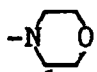, $CO_2$-$C_1$-$C_4$-alkyl, $C_1$-$C_7$-alkoxy, and aryl, as defined above;

-CO-$C_1$-$C_4$-alkyl ; -$CO_2$-$C_1$-$C_7$-alkyl ; -CO-NH-$C_1$-$C_7$-alkyl ; -$SO_2$-$C_1$-$C_7$-alkyl ; -CHO ; and -CO-aryl, -CO-NH-aryl or -$SO_2$-aryl, where aryl is as defined above ; or, when attached to a quaternized $sp^3$ hybridized nitrogen in the heterocyclic ring, are independently selected from the group consisting of $C_1$-$C_7$-alkyl and mono-substituted $C_1$-$C_4$-alkyl, where the substituent is independently selected from the group consisting of hydroxyl, -$CO_2$H,

-$CO_2$-$C_1$-$C_4$- alkyl, -$SO_3$H, -$SO_2NH_2$, $C_1$-$C_4$-alkoxy, di-$C_1$-$C_4$-alkylamino, -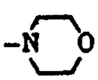, and aryl, as defined above; or, are

alternatively -$(CH_2)_q$- or -$(CH_2)_2$-O-$(CH_2)_2$- and form a quaternary spirocyclic ring with the $sp^3$ hybridized N-atom, wherein q is 3-to-6 ; or, when attached to a quaternized $sp^2$ hybridized nitrogen in the heterocyclic ring, are independently selected from the group consisting of $C_1$-$C_7$-alkyl and mono-substituted $C_1$-$C_4$-alkyl, where the substituent is independently selected from the group consisting of hydroxyl, $C_1$-$C_4$-alkoxy, -$CO_2$H and -$CO_2$-$C_1$-$C_4$-alkyl ;

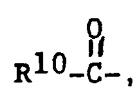

where $R^{10}$ is $C_1$-$C_7$-alkyl ;

hydrogen ;

Het, as defined above ;

aryl, as defined above ;

mono-substituted $C_1$-$C_6$-alkyl, wherein the substituent is selected from the group consisting of aryl, as defined above ; Het, as defined above ; hydroxyl ; $C_1$-$C_4$-alkoxy ; $C_3$-$C_7$-cycloalkyl ; amino ; mono- or di-$C_1$-$C_4$-alkyl-amino ; Het-$C_1$-$C_4$-alkyl, wherein Het is as defined above ; aryl or aryl-$C_1$-$C_4$-alkyl, wherein aryl is as defined above ; -$CO_2$H ;

-$CO_2R^{11}$, where $R^{11}$ is $C_1$-$C_5$-alkyl, aryl, as defined above, Het, as defined above, mono-substituted $C_2$-$C_5$-alkyl, wherein the substituent is hydroxyl, $C_1$-$C_4$-alkoxy, $C_1$-$C_5$-alkyl-$CO_2$-, $C_1$-$C_5$-alkyl-CONH-, H-CONH-, amino, mono- or dialkylamino or halo ;

-$CONH_2$ ; -CONH-$R^{11}$ or -$S(O)_n$-$R^{11}$, wherein n is 0-to-2 and $R^{11}$ is as defined above ; -NH-CO-$R^{11}$, where $R^{11}$ is as defined above ; -NH-aryl, -NH-$CH_2$-aryl or -CO-aryl, where aryl is as defined above ; and -NH-Het, -NH-$CH_2$-Het or -CO-Het, where Het is as defined above ;

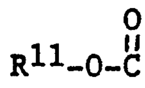 or 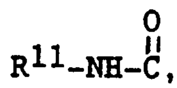

where $R^{11}$ is as defined above ; or

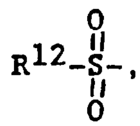

where $R^{12}$ is independently selected from the definitions of $R^{11}$, $C_6$-or-$C_7$-alkyl, or Het, as defined above ;
B and D are independently

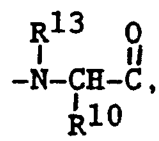

where $R^{13}$ is hydrogen, $C_1$-$C_5$-alkyl, or -$CH_2$-aryl, wherein aryl is as defined above ; and $R^{10}$ is as defined above;

or 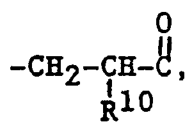

where $R^{10}$ is as defined above ; or either B or D, but not both simultaneously, is absent ;

$R^1$ is hydrogen ;
$C_3$-$C_6$-alkyl ;
aryl, as defined above ;
unsubstituted, mono-, di- or trisubstituted $C_3$-$C_7$-cycloalkyl, where the substituent(s) is/are selected from the group consisting of $C_1$-$C_4$-alkyl, trifluoromethyl, hydroxyl, $C_1$-$C_4$-alkoxy and halo ; or unsubstituted or 4-monosubstituted 1,3-dithiolan-2-yl or unsubstituted or 4-mono-substituted 1,3-dithian-2-yl, where the substituent is -$(CH_2)_m$-aryl, where m and aryl are as defined above ;

$R^2$ is hydrogen, $C_1$-$C_7$-alkyl, $C_2$-$C_7$-alkenyl, phenyl or $C_1$-$C_3$-alkyl-phenyl ;

X is -$CH_2$- ; -O- ; -$CH_2CH_2$- ; -CH=CH- ; -$CH_2$NH- : -CHO-, where $R^{14}$ is hydrogen or $C_1$-$C_7$-alkyl ; $R^{14}$ -$CH_2$CH-, where $R^{15}$ is $C_1$-$C_6$ alkyl, $R^{15}$ hydroxyl, $C_1$-$C_4$ alkoxyl, $C_1$-$C_6$ acyloxy, amino, mono-$C_1$-$C_6$-alkylamino, di-$C_1$-$C_6$-alkylamino, amino-$C_1$-$C_5$-alkyl, mono-$C_1$-$C_6$-alkylamino- $C_1$-$C_6$-alkyl, $C_1$-$C_4$ alkoxylthio-$C_1$-$C_4$-alkyl, fluoro, carboxy, carboxy-$C_1$-$C_6$-alkylamido, aryl or aryl $C_1$-$C_4$ alkoxyl, where aryl is as defined above ;

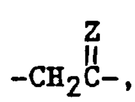

where Z is oxo, -$OCH_2$-, $C_1$-$C_6$ alkyl imino, methylene, $C_1$-$C_6$ alkylmethylene, ;

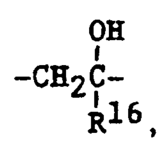

where $R^{16}$ is aminomethyl, mono or di-$C_1$-$C_5$-alkyl-aminomethyl, 4-morpholino, 1-pyrrolidinylmethyl, 1-piperidi-

nylmethyl ;

$R^3$ is hydrogen ; aryl or -CO-aryl, where aryl is as defined above ; -CO-Het, where Het is as defined above; $-CO_2H$ ; $-CO-NH-R^{11}$ or $-CO-N(R^{13})-R^{11}$, where $R^{13}$ and $R^{11}$ are as defined above ; or unsubstituted or mono-substituted $C_1$-$C_8$-alkyl or -CO-$C_1$-$C_8$-alkyl, $C_2$-$C_8$-alkenyl, $C_3$-$C_7$-cycloalkyl or $C_5$-$C_7$-cycloalkenyl, where the substituent on the $C_1$-$C_8$-alkyl, $C_2$-$C_8$-alkenyl, $C_3$-$C_7$-cycloalkyl or $C_5$-$C_7$-cycloalkenyl is selected from the group consisting of $C_1$-$C_7$-alkyl, $C_3$-$C_7$-cyclo-alkyl, hydroxyl, halo, -CHO, $-CO_2H$, $-CO_2R^{11}$ or $-CONH-R^{11}$ or $-NHCO-R^{11}$, wherein $R^{11}$ is as defined above, $-O-CO-R^{12}$, wherein $R^{12}$ is as defined above, amino, mono- or di-$C_1$-$C_4$-alkylamino, mono- amino-$C_1$-$C_4$-alkylamino, -$NHR_{11}$, $-N(R^{13})-CO-R^{12}$ or $-CON(R^{13})-R^{11}$, wherein $R^{13}$, $R^{12}$ and $R^{11}$ are as defined above,

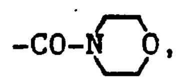

and aryl, as defined above ;

$R^4$ is when X is

$$-\underset{\mid}{\overset{}{C}}HO-,\ -O-,\ \text{or}\ -CHNH-,$$
$$R^{14}$$

hydrogen ;

aryl, as defined above ;

Het, as defined above ;

unsubstituted or monosubstituted $C_1$-$C_5$-alkyl, where the substituent is selected from the group consisting of hydroxyl ; $-CO_2H$ ; $-CO_2R^{11}$ or $-CONH-R^{11}$, wherein $R^{11}$ is as defined above ; $-O-COR^{12}$, wherein $R^{12}$ is as defined above ; amino ; mono- or di-$C_1$-$C_4$-alkylamino ; $-N(R^{13})$- $COR^{12}$ or $-CON(R^{13})-R^{11}$, where $R^{13}$, $R^{12}$ and $R^{11}$ are as defined above ;

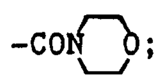

aryl, as defined above ; Het, as defined above ; and $-^{+}N(R^5)_2R^9$ $A^-$ or

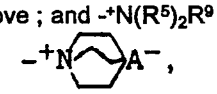

wherein $R^5$, $R^9$ and $A^-$ are as defined above ;

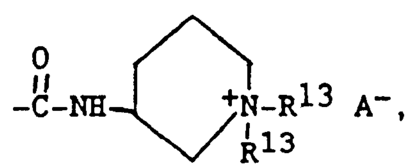

where $R^{13}$ and $A^-$ are as defined above ; or

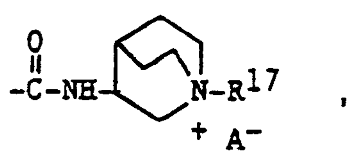

where $R^{17}$ is $C_1$-$C_4$-alkyl, $C_5$-$C_6$-cycloalkyl, carboxy-$C_1$-$C_4$- alkyl, carbo-$C_1$-$C_4$-alkoxy-$C_1$-$C_4$-alkyl, or $-CH_2$-aryl or $-CH_2$-Het, wherein aryl, Het and $A^-$ are as defined above ; or

$R^4$ is when X is $-CH_2-$, $-CH_2CH_2-$, $-CH=CH-$, $-CH2CH(R15)-$, $-CH2C(=Z)-$, or $-CH_2C(OH)(R^{16})-$

hydrogen ; $C_1$-$C_7$-alkyl ; aryl, as defined above ; Het, as defined above ; $-CO_2H$ ; $-CO_2R^{11}$ or $-CONH-R^{11}$, where $R^{11}$ is as defined above ; hydroxyl ; $-O-COR^{12}$, where $R^{12}$ is as defined above ; amino ; mono- or di-$C_1$-$C_4$-alkylamino ; $-N(R^{13})-COR^{12}$ or $-CON(R^{13})-R^{11}$, where $R^{13}$,

$R^{12}$ and $R^{11}$ are as defined above; 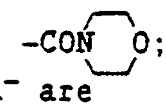; $-{}^{+}N(R^5)_2R^9$ $A^-$, where $R^5$, $R^9$ and $A^-$ are as defined above; 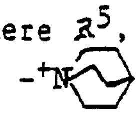 $A^-$;

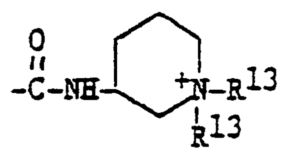 $A^-$, 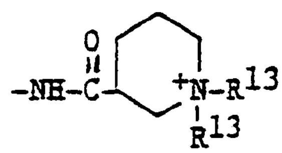 $A^-$,

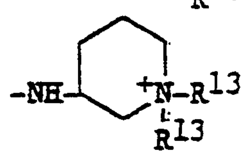 $A^-$, where $R^{13}$ and $A^-$ are as defined above;

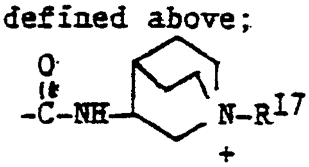 $A^-$, 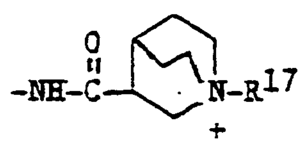 $A^-$,

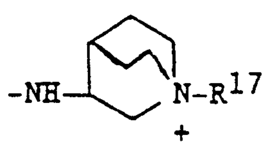 $A^-$,

where $R^{17}$ is as defined above ; substituted $C_1$-$C_5$-alkyl, wherein the substituent is selected from the group consisting of hydroxyl, $-CO_2H$, $-CO_2R^{11}$ or $-CONH-R^{11}$, where $R^{11}$ is as defined above, $-O-COR^{12}$, where $R^{12}$ is as defined above, amino, mono- or di-$C_1$-$C_4$-alkylamino, $-N(R^{13})-COR^{12}$, or $-CON(R^{13})-R^{11}$, where $R^{13}$, $R^{12}$ and $R^{11}$ are as defined above, 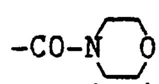, aryl, as defined above, Het, as defined above, and $-{}^{+}N(R^5)_2R^9$ $A^-$ or 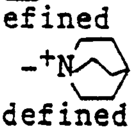 $A^-$, where $R^5$, $R^9$ and $A^-$ are as defined above,

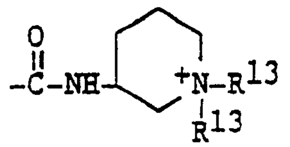 $A^-$, 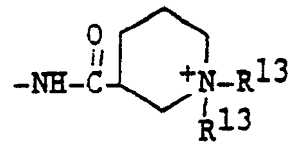 $A^-$,

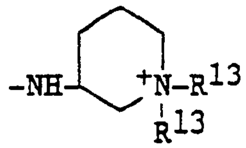 $A^-$, where $R^{13}$ and $A^-$ are as defined above,

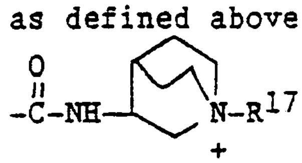 $A^-$, 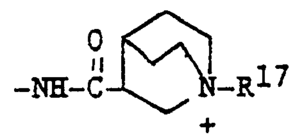 $A^-$,

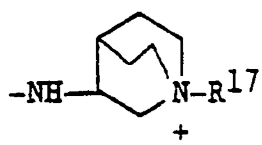 $A^-$, where $R^{17}$ is as defined above ;

and pharmaceutically-acceptable salts thereof.

In the peptides of the present invention, the components having asymmetric centers occur as racemates, racemic mixtures and as individual diastereomers, with all isomeric forms generally being included in the present invention. In particular, asymmetric carbon atoms at the 2, 3 and 4 positions in peptides of formula I preferably have an S configuration.

When any variable (e.g., aryl, Het, m, $R^5$, $R^6$, $R^7$, $R^8$, $R^9$, $R^{10}$, $R^{11}$, $R^{12}$, $R^{13}$, $A^-$, etc.) occurs more than one time in any variable or in formula I, its definition on each ocurrence is independent of its definition at every other occurrence.

As used herein, "alkyl" is intended to include both branched- and straight-chain saturated aliphatic hydrocarbon groups having the specified number of carbon atoms (Me is methyl, Et is ethyl) ; "alkoxy" represents an alkyl group of indicated number of carbon atoms attached through an oxygen bridge ; and "$C_3$-$C_7$-cycloalkyl" is intended to include saturated ring groups, such as cyclopropyl, cyclobutyl, cyclopentyl, cyclohexyl and cycloheptyl. "Alkanoyl" is intended to include those alkylcarbonyl groups of specified number of carbon atoms, which are exemplified by formyl, acetyl, propanoyl and butanoyl ; "alkenyl" is intended to include hydrocarbon chains of either a straight- or branched- configuration and one unsaturation, which may occur at any point along the chain, such as ethenyl, propenyl, butenyl, pentenyl, and the like, and includes E and Z forms, where applicable ; and "arylalkyl" represents aryl groups as herein defined which are attached through a straight- or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo, and "counterion" is used to represent a small, single negatively-charged specie, such as chloride, bromide, hydroxide, nitrate, acetate, benzoate, perchlorate, benzene sulfonate, tartrate, hemitartrate, maleate, and the like.

As used herein, with exceptions as noted, "aryl" is intended to mean phenyl (Ph), which is optionally-substituted by from one- to three- members independently selected from the group consisting of $C_1$-$C_7$-alkyl, amino (Am), mono- or di-$C_1$-$C_4$-alkylamino, phenyl-$C_1$-$C_4$-alkyl, amino-$C_1$-$C_4$-alkyl, hydroxy- $C_1$-$C_4$-alkyl, mono- or di-$C_1$-$C_4$-alkyamino-$C_1$- $C_4$-alkyl, hydroxyl, guanidyl, guanidyl-$C_1$-$C_4$- alkyl, $C_1$-$C_4$-alkoxy, $CF_3$, halo, CHO, $CO_2H$, $CONH_2$,

CONH-$C_1$-$C_4$-alkyl, CON($C_1$-$C_4$-alkyl$)_2$, CO-$C_1$-$C_4$-alkyl, $(CH_2)_m$-$^+N(R^5)_2R^6$ $A^-$, -CO-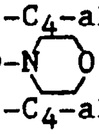, -$(CH_2)_m$-$^+$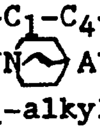 $A^-$, $CO_2$-$C_1$-$C_4$-alkyl, -$CO_2$-$C_1$-$C_4$-alkoxy-$C_2$-$C_4$-alkyl or $NR^7R^8$, wherein $R^5$, $R^6$, $R^7$, $R^8$ and m are as defined above and $A^-$ is counterion, as defined herein. "Aroyl" is intended to include those aryl-carbonyl groups which are exemplified by phenoyl.

The term "Het", as used herein, represents a 5-to-7-membered monocyclic or 7-to-10-membered bicyclic hetrocyclic ring which is either saturated or unsaturated, and which consists of carbon atoms and one or two heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined 5-to-7-membered monocyclic heterocyclic rings is fused to a benzene ring. Heterocycles which contain nitrogen are preferred. In the case of a heterocyclic ring containing one or more nitrogen atoms, the point of attachment may be at one of the nitrogen atoms, or at any carbon atom. Examples of such heterocyclic elements include piperidyl, piperidinyl, piperazinyl, 2-oxopiperazinyl, 2-oxopyrolodinal, 2-oxopiperidinyl, 2-oxoazepinyl, azepinyl, pyrryl, pyrrolinyl, 4-piperidonyl, pyrrolidinyl, pyrazolyl, pyrazolinyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, benzothiazolyl, benzoxazolyl, furyl, thienyl, benzothienyl, thiamorpholinyl, thiamorpholinyl sulfoxide, and thiamorpholinyl sulfone. The heterocyclic moiety is further optionally-substituted by from one- to four-members independently selected according to whether the substituent in on a carbon atom, an $sp^3$ hybridized nitrogen, a quaternized $sp^3$ nitrogen or a quaternized $sp^2$ nitrogen from the various groups defined above, and including spiro quaternary species.

The following additional abbreviations have also been used herein :

| Abbreviated Designation | Amino Acid/Residue |
|---|---|
| ACHPA | (3S,4S)-4-amino-5-cyclohexyl-3-hydroxypentanoic acid |
| Ala | L-alanine |
| Arg | L-arginine |
| Cys | cysteine |
| Gly | L-glycine |
| His | D- or L-histidine |
| HomoPhe | homologated phenylalanine |
| HomoTrp | homologated tryptophan |
| HomoTyr | homologated tyrosine |
| Ile | L-isoleucine |
| Leu | L-leucine |
| Lys | L-lysine |
| Met | L-methionine |
| Nle | norleucine |
| Nva | norvaline |
| Orn | L-ornithine |
| (p-MeO)Phe | L-*para*-methoxyphenylalanine |
| Phe | L-phenylalanine |
| Pro | proline |
| Sar | L-sarcosine (N-methylglycine) |
| Ser | L-serine |
| Sta | statine |
| Thr | L-threonine |
| **Abbreviated Designation** | **Amino Acid/Residue** |
| Trp | L-tryptophan |
| Tyr | L-tyrosine |
| Val | L-valine |
| | **Protecting Group** |
| BOC | *t*-butyloxycarbonyl |
| CBZ | benzyloxycarbonyl(carbobenzoxy) |
| DNP | 2,4-dinitrophenyl |
| IPOC | isopropoxycarbonyl |
| ETOC | ethoxycarbonyl |
| | **Activating Group** |
| HBT(HOBt) | 1-hydroxybenzotriazole hydrate |
| HOSU | N-hydroxysuccinimide |
| | **Condensing Agent** |

| | |
|---|---|
| DCCI (DCC) | dicyclohexylcarbodiimide |
| DPPA | diphenylphosphorylazide |
| EDC | 1-(3-dimethylaminopropyl)-3-ethyl-carbodiimide hydrochloride |
| BOP | benzotriazol-1-yloxytris(dimethyl-amino)phosphonium hexafluoro-phosphate |
| | Reagent |
| $(BOC)_2O$ | di-t-butyl dicarbonate |
| DIBAL | diisobutylaluminum hydride |
| DIPEA | diisopropylethylamine |
| DMAP | 4-(dimethylamino)pyridine |
| TEA | triethylamine |
| Abbreviated Designation | Reagent |
| TFA | trifluoroacetic acid |
| LAH | lithium aluminum hydride |
| LDA | lithium diisopropylamide |
| MCPBA | 3-chloroperoxybenzoic acid |
| NMM | N-methyl morpholine |
| PPTS | pyridinium para-toluenesulfonate |
| TBAF | tetra-n-butylammonium fluoride |
| | Solvent |
| HOAc (AcOH) | acetic acid |
| $CH_2Cl_2$ | dichloromethane |
| DMF | dimethylformamide |
| DMSO | dimethyl sulfoxide |
| EtOAc | ethyl acetate |
| EtOH | ethanol |
| $Et_2O$ | ether |
| MeOH | methanol |
| THF | tetrahydrofuran |

The novel renin inhibitory peptides of the present invention may be generalized and alternately described in terms of common amino acid components and closely-related analogs thereof, in accordance with formula I, wherein :

A, $R^1$, $R^2$, $R^3$, $R^4$ and X are as defined under Formula I ;

B is Absent, Ala, Leu, Phe, HomoPhe, (p-MeO)Phe,

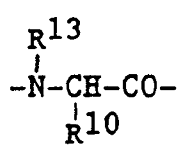, Tyr, Trp, HomoTrp, 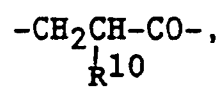,

or where $R^{10}$ and $R^{13}$ are as defined above ;

D is Absent, Ala, Ser, Met, Thr, Phe, Tyr, Trp,

Nle, His, Lys, Orn, Arg or Val, 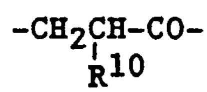

or 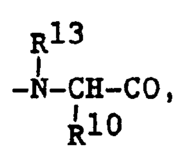,

where $R^{10}$ and $R^{13}$ are as defined above, such that B and D are not simultaneously absent.

In terms of substrate analogy, a unique aspect and essential feature of the present invention is the substitution of the

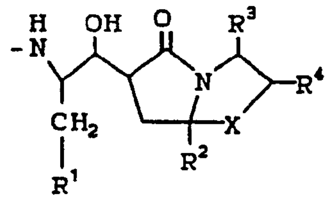

component for the triple amino acid sequence, $Leu^{10}$-$Val^{11}$-$Ile^{12}$ in the endogenous human renin substrate,

| | | | | | | |
|---|---|---|---|---|---|---|
| 7 | 8 | 9 | 10 | 11 | 12 | 13 |
| (Pro | Phe | His | Leu | Val | Ile | His) . |

It will be understood that closely-related analogs of the above common amino acids, for example, aliphatic amino acids in addition to Ala, Val, Leu, and Ile, such as α-aminobutyric acid (Abu), derivatives of amino acids having heteroatom- containing side chains, substituted phenyl derivatives of Phe, and $N^{\alpha}$-methyl amino acids, are included in the broad description of the novel inhibitory peptides of the present invention represented by Formula I and related definitions.

Preferred renin-inhibitory peptides are those wherein A is $R^{10}$-CO-, $R^{11}$-O-CO-, $R^{11}$-$SO_2$-, $R^{12}$-$SO_2$, or $R^{11}$-NH-CO-, wherein $R^{10}$, $R^{11}$ and $R^{12}$ are as defined above ; B is absent (when D is present), L-phenylalanyl or derivatives thereof substituted on the aromatic ring, or is

$-CH_2-CH-CO-$ ;
$CH_2Ph$

D is absent (when B is present), L-histidyl, N-α-methyl-L-histidyl, L-valinyl or L-nor-leucinyl ; $R^1$ is cyclohexyl; $R^2$ is hydrogen or methyl ; $R^3$ is n-propyl, 2-methylpropyl, or hydrogen ; and $R^4$ is $-CH_2-^{+}N(R^5)_2R^9\ A^-$ where $R^5$, $R^9$ and $A^-$ are defined as above, or hydrogen, when X is $-CH_2O-$, or $R^4$ is $-^{+}N(R^5)_2R^9\ A^-$ or $-CH_2-^{+}N(R^5)_2R^9\ A^-$, when X is $-CH_2CH_2-$.

Representative preferred renin-inhibitory peptides of the present invention include the following compounds having the structure :

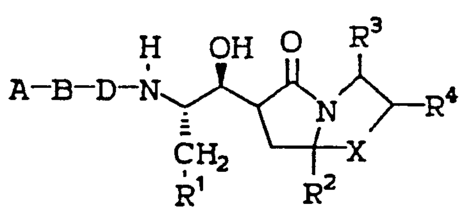

wherein, in the structures:

| | A | B | D | R1 | R2 | X | R3 | R4 |
|---|---|---|---|---|---|---|---|---|
| 1. | Boc | Phe | His | cyclo-$C_6H_{11}$ | H | $-CH_2O-$ | H | H |
| | " | " | " | " | " | " | $-CH_2CH=CH_2$ | " |
| | " | " | " | " | " | " | $-CH_2CH_2CH_3$ | " |
| | " | " | " | " | " | " | $-CH(CH_3)CH_2CH_3$ | " |
| 5. | " | " | " | " | " | " | $-CH(CH_3)CH=CH_2$ | " |
| | " | " | " | " | " | " | $-CH_2C(CH_3)=CH_2$ | " |
| | " | " | " | " | " | " | $-CH_2CH(CH_3)_2$ | " |
| | " | " | " | " | " | " | -cyclo-$C_5H_7$ | " |
| | " | " | " | " | " | " | -cyclo-$C_5H_9$ | " |

| | A | B | D | $R^1$ | $R^2$ | X | $R^3$ | $R^4$ |
|---|---|---|---|---|---|---|---|---|
| 10. | Boc | Phe | His | cyclo-$C_6H_{11}$ | H | $-CH_2O-$ | $-CH_2CH_2CH_3$ | H |
| | " | " | " | " | " | $-CH_2CH_2-$ | " | " |
| | " | " | " | " | " | $-CH(CH_3)O-$ | " | " |
| | " | " | " | " | " | $-CH_2CO-$ | " | " |
| | " | " | " | " | " | $-CH_2CH(OH)-$ | " | " |
| 15. | " | " | " | " | " | $-CH_2CH-$<br>$\vert$<br>$N(CH_3)_2$ | " | " |
| | " | " | " | " | " | $-CH_2NH-$ | " | " |
| | " | " | " | " | $CH_3$ | $-CH_2O-$ | H | " |
| | " | " | " | " | " | " | $-CH_2CH_2CH_3$ | " |
| 20. | " | " | " | " | " | " | H | $-CH_2NH_2$ |
| | " | " | " | " | " | $-CH_2CH_2-$ | $-CH_2CH_2CH_3$ | H |
| | " | " | " | " | " | $-CH_2CO-$ | " | " |
| | " | " | " | " | " | $-CH_2CH(OH)-$ | " | " |
| | " | " | " | " | " | $-CH_2CH-$<br>$\vert$<br>$N(CH_3)_2$ | " | " |
| 25. | " | " | " | " | " | " | H | " |
| | " | " | " | " | " | $-CH_2CH-$<br>$\vert$<br>$NH_2$ | " | " |
| | " | " | " | " | " | $-CH_2CO-$ | " | $-CH_2N(CH_3)_2$ |
| | " | " | " | " | " | $-CH_2CH(OH)-$ | " | " |
| | " | " | " | " | " | $-CH_2CO-$ | " | $-CH_2N(CH_3)_3^+$<br>$OAc^-$ |
| 30. | " | " | " | " | " | $-CH_2CH(OH)-$ | " | " |

| | A | B | D | $R^1$ | $R^2$ | X | $R^3$ | $R^4$ |
|---|---|---|---|---|---|---|---|---|
| 31. | Boc | Phe | His | cyclo-$C_6H_{11}$ | H | -O- | $-CH_2CH_2CH_3$ | H |
| | " | " | " | " | " | " | -Phenyl | " |
| | " | " | " | " | $CH_3$ | " | $-CH_2CH_2CH_3$ | " |
| | " | " | " | " | " | $-CH_2CO-$ | $-(CH_2)_2N(CH_3)_2$ | " |
| 35. | " | " | " | " | " | " | $-CH_2CH_2OH$ | " |
| | Etoc | Phe | His | cyclo-$C_6H_{11}$ | H | $-CH_2O-$ | H | " |
| | " | " | " | " | " | " | $-CH_2CH=CH_2$ | " |
| | " | " | " | " | " | " | $-CH_2CH_2CH_3$ | " |
| | " | " | " | " | " | " | $-CH(CH_3)CH_2CH_3$ | " |
| 40. | " | " | " | " | " | " | $-CH(CH_3)CH=CH_2$ | " |
| | " | " | " | " | " | " | $-CH_2C(CH_3)=CH_2$ | " |
| | " | " | " | " | " | " | $-CH_2CH(CH_3)_2$ | " |
| | " | " | " | " | " | " | -cyclo-$C_5H_7$ | " |
| | " | " | " | " | " | " | -cyclo-$C_5H_9$ | " |
| 45. | " | " | " | " | " | $-CH_2O-$ | $-CH_2CH_2CH_3$ | " |
| | " | " | " | " | " | $-CH_2CH_2-$ | " | " |
| | " | " | " | " | " | $-CH(CH_3)O-$ | " | " |
| | " | " | " | " | " | $-CH_2CO-$ | " | " |
| | " | " | " | " | " | $-CH_2CH(OH)-$ | " | " |
| 50. | " | " | " | " | " | $-CH_2CH(N(CH_3)_2)-$ | " | " |

| | A | B | D | $R^1$ | $R^2$ | X | $R^3$ | $R^4$ |
|---|---|---|---|---|---|---|---|---|
| 51. | Etoc | Phe | His | cyclo-$C_6H_{11}$ | H | $-CH_2CH(NH_2)-$ | H | H |
| | " | " | " | " | $CH_3$ | $-CH_2O-$ | " | " |
| | " | " | " | " | " | " | $-CH_2CH_2CH_3$ | " |
| 55. | " | " | " | " | " | " | H | $-CH_2NH_2$ |
| | " | " | " | " | " | $-CH_2CH_2-$ | $-CH_2CH_2CH_3$ | H |
| | " | " | " | " | " | $-CH_2CO-$ | " | " |
| | " | " | " | " | " | $-CH_2CH(OH)-$ | " | " |
| | " | " | " | " | " | $-CH_2CH(N(CH_3)_2)-$ | " | " |
| 60. | " | " | " | " | " | " | H | " |
| | " | " | " | " | " | $-CH_2NH-$ | " | " |
| | " | " | " | " | " | $-CH_2CO-$ | " | $-CH_2N(CH_3)_2$ |
| | " | " | " | " | " | $-CH_2CH(OH)-$ | " | " |
| | " | " | " | " | " | $-CH_2CO-$ | " | $-CH_2N(CH_3)_3^+$ $OAc^-$ |
| 65. | " | " | " | " | " | $-CH_2CH(OH)-$ | " | " |

More preferred renin-inhibitory peptides include those wherein in the structures :

| | A | B | D | $R^1$ | $R^2$ | X | $R^3$ | $R^4$ |
|---|---|---|---|---|---|---|---|---|
| 1. | Etoc | Phe | His | cyclo-$C_6H_{11}$ | H | $-CH_2O-$ | H | $-CH_2NH_2$ |
| | " | " | " | " | " | " | " | $-CO_2H$ |
| | " | " | " | " | " | " | " | $-CONHCH_2CH_2N(CH_3)_2$ |
| | " | " | " | " | " | " | " | $-CH_2NHCO-$ 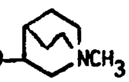 + OAc⁻ |
| 5. | " | " | " | " | " | " | " | $-CONH-$ 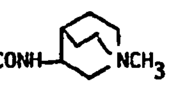 + OAc⁻ |
| | " | " | " | " | $CH_3$ | " | " | $-CH_2NH_2$ |
| | " | " | " | " | " | " | " | $-CO_2H$ |
| | " | " | " | " | " | " | " | $-CONHCH_2CH_2N(CH_3)_2$ |
| | " | " | " | " | " | " | " | $-CH_2NHCO-$ 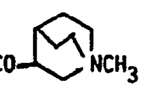 + OAc⁻ |
| 10. | " | " | " | " | " | " | " | $-CONH-$ 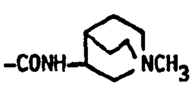 + OAc⁻ |

| | A | B | D | $R^1$ | $R^2$ | X | $R^3$ | $R^4$ |
|---|---|---|---|---|---|---|---|---|
| 11. | Etoc | Phe | Nle | cyclo-$C_6H_{11}$ | H | $-CH_2O-$ | H | H |
| | " | " | " | " | " | " | " | $-CH_2NH_2$ |
| | " | " | " | " | " | " | " | $-CO_2H$ |
| | " | " | " | " | " | " | " | $-CONHCH_2CH_2N(CH_3)_2$ |
| 15. | " | " | " | " | " | " | " | 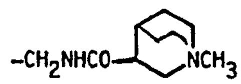 + $OAc^-$ |
| | " | " | " | " | " | " | " | 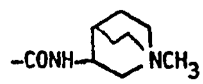 + $OAc^-$ |
| | " | " | " | " | $CH_3$ | " | " | $-CH_2NH_2$ |
| | " | " | " | " | " | " | " | $-CO_2H$ |
| | " | " | " | " | " | " | " | $-CONHCH_2CH_2N(CH_3)_2$ |
| 20. | " | " | " | " | " | " | " | 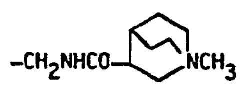 + $OAc^-$ |
| | " | " | " | " | " | " | " | 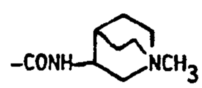 + $OAc^-$ |

| | A | B | D | $R^1$ | $R^2$ | X | $R^3$ | $R^4$ |
|---|---|---|---|---|---|---|---|---|
| 22. | Etoc | Phe | His | cyclo-$C_6H_{11}$ | H | $-CH_2O-$ | $-CH_2CH_2CH_3$ | $-CH_2NH_2$ |
| | " | " | " | " | " | " | " | $-CO_2H$ |
| | " | " | " | " | " | " | " | $-CONHCH_2CH_2N(CH_3)_2$ |
| 25. | " | " | " | " | " | " | " | 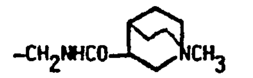 + OAc⁻ |
| | " | " | " | " | " | " | " | 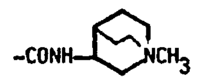 + OAc⁻ |
| | " | " | " | " | " | " | " | $-CH_2NH_2$ |
| | " | " | " | " | " | " | " | $-CO_2H$ |
| | " | " | " | " | " | " | " | $-CONHCH_2CH_2N(CH_3)_2$ |
| 30. | " | " | " | " | " | " | " | 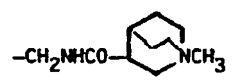 + OAc⁻ |
| | " | " | " | " | " | " | " | 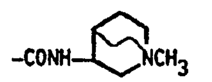 + OAc⁻ |

Most preferred renin-inhibitory peptides include those wherein in the structures :

| | A | B | D | R1 | R2 | X | R3 | R4 |
|---|---|---|---|---|---|---|---|---|
| 1. | 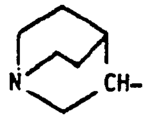 | Phe | His | cyclo-$C_6H_{11}$ | H | $-CH_2O-$ | $-CH_2CH_2CH_3$ | H |
| | 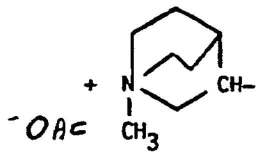 | " | " | " | " | " | " | " |
| | 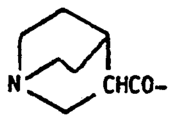 | " | " | " | " | " | " | " |
| | 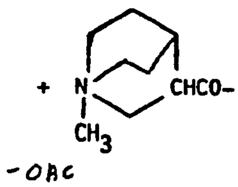 | " | " | " | " | " | " | " |

| | A | B | D | R1 | R2 | X | R3 | R4 |
|---|---|---|---|---|---|---|---|---|
| 5. | 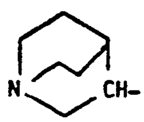 | Phe | Nle | cyclo-$C_6H_{11}$ | H | $-CH_2O-$ | $-CH_2CH_2CH_3$ | H |
| | 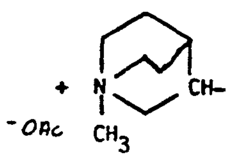 | " | " | " | " | " | " | " |
| | 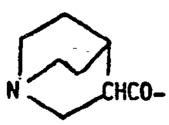 | " | " | " | " | " | " | " |
| | 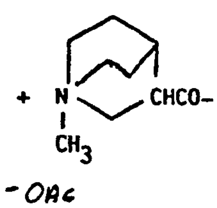 | " | " | " | " | " | " | " |

| | A | B | D | R1 | R2 | X | R3 | R4 |
|---|---|---|---|---|---|---|---|---|
| 9. | 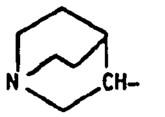 | Phe | His | cyclo-$C_6H_{11}$ | H | $CH_2CH_2$ | $-CH_2CH_2CH_3$ | H |
| | 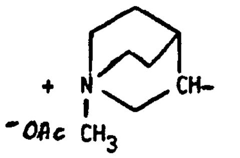 | " | " | " | " | " | " | " |
| | 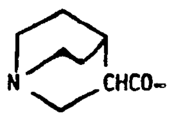 | " | " | " | " | " | " | " |
| | 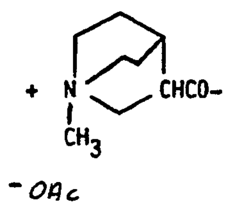 | " | " | " | " | " | " | " |

| | A | B | D | R1 | R2 | X | R3 | R4 |
|---|---|---|---|---|---|---|---|---|
| 13. | 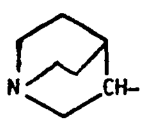 | Phe | Nle | cyclo-$C_6H_{11}$ | H | $CH_2CH_2$ | $-CH_2CH_2CH_3$ | H |
| | 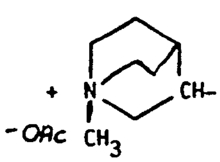 | " | " | " | " | " | " | " |
| | 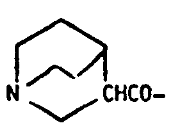 | " | " | " | " | " | " | " |
| | 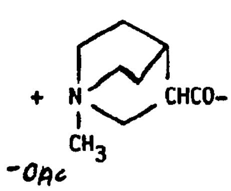 | " | " | " | " | " | " | " |

| | A | B | D | $R^1$ | $R^2$ | X | $R^3$ | $R^4$ |
|---|---|---|---|---|---|---|---|---|
| 17. | 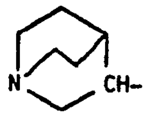 | Phe | His | cyclo-$C_6H_{11}$ | H | $CH_2CH(OH)$ | $-CH_2CH_2CH_3$ | H |
| | 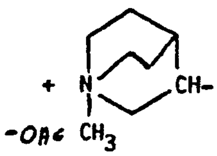 | " | " | " | " | " | " | " |
| | 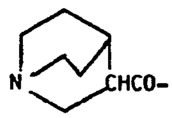 | " | " | " | " | " | " | " |
| | 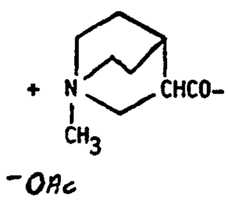 | " | " | " | " | " | " | " |

| | A | B | D | $R^1$ | $R^2$ | X | $R^3$ | $R^4$ |
|---|---|---|---|---|---|---|---|---|
| 21. | 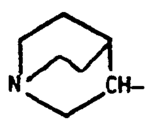 | Phe | Nle | cyclo-$C_6H_{11}$ | H | $CH_2CH(OH)$ | $-CH_2CH_2CH_3$ | H |
| | 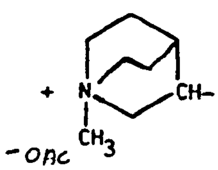 | " | " | " | " | " | " | " |
| | 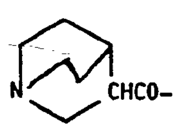 | " | " | " | " | " | " | " |
| | 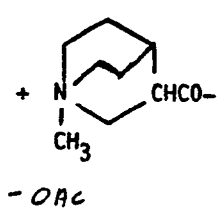 | " | " | " | " | " | " | " |

The pharmaceutically-acceptable salts of the peptides of formula I (in the form of water- or oil-soluble or dispersible products) include the conventional non-toxic salts or the quaternary ammonium salts of these peptides which are formed, e.g., from inorganic or organic acids or bases. Examples of such acid addition salts include acetate, adipate, alginate, aspartate, benzoate, benzene- sulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methane- sulfonate, 2-naphthalenesulfonate, nicotinate, oxalate, pamoate, pectinate, persulfate, 3-phenyl- propionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides ; dialkyl sulfates like dimethyl, diethyl, dibutyl ; and diamyl sulfates, long chain halides such as decyl, lauryl, myristyl and stearyl chlorides, bromides and iodides, aralkyl halides like benzyl and phenethyl bromides and others.

Chemical synthesis of the compounds with the general structure given in formula I may be accomplished in several ways as illustrated by the following generalized procedures (wherein "ACHP", an abbreviation of 2-Amino-3-Cyclohexyl-1-HydroxyPropyl, is used in describing the structural segment which joins the A-B-D and

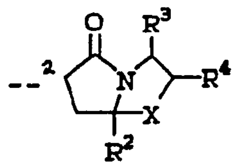

bicyclic lactam portions of the invention as shown in formula I. The ACHP segment is connected through the 1-position of the propyl chain to the 2-position of the bicyclic lactam moiety, and through the amino group to the A-B-D-segment).

Method A :

Step A1. A bicyclic lactam derivative having the structure :

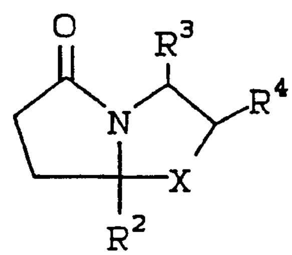

wherein $R^2$ - $R^4$ and X are defined above unless otherwise indicated, is obtained through commercial sources or is prepared using well known chemical methods for preparation of compounds of this type (see Example 1);

Step A2. The enolate of the bicyclic lactam derivative is generated, such as by using LDA as the base, and is added to a nitrogen-protected (e.g., N-Boc or N-CBZ protected) α-amino-aldehyde (e.g., N-tert-butyloxycarbonyl-cyclohexylalaninal) to give a Boc- or CBZ- protected amino alcohol derivative (see Example 2) ;

Step A3. The nitrogen protecting group of the amino alcohol derivative from step A2 is removed (e.g., by hydrogenolysis for CBZ protection, or TFA treatment for Boc protection) and the amine is coupled using standard peptide methodology to one or two amino acids, or to an appropriate carboxylic acid, the structure(s) of which is/are described by A, B, and D in the general formula I (e.g. see Example 3A steps 1 and 2, and Example 3B) ; and

Step A4. Removal of any protecting groups which may have been used, e.g., on the lactam substituent(s) ($R^3$, $R^4$ and X), or on the amino acid side chains (see Example 3A, step 3), gives the final products.

Method B :

Steps A1 and A2 are as followed.

Step B3. Modification of the lactam substituent(s) ($R^3$, $R^4$ and X in generic formula I) is/are performed, as shown in Examples 2II-2XX.

Then Steps A3 and A4 are followed.

Method C :

Steps A1, A2 and A3 are followed.

Step C4 : modification of the lactam substituent(s) ($R^3$, $R^4$ and X in generic formula I) is/are performed, as shown in Examples 3YY-3CCC.

Step A4 is then followed.

The novel peptides of the present invention possess a high degree of activity in treating renin-associated hypertension, hyperaldosteronism and/or congestive heart failure in humans, as well as in other warm-blooded animals such as mice, rats, horses, dogs and cats.

In addition, the peptides of the present invention have utility in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Treating AIDS, preventing infection by HIV or treating infection by HIV, is defined as including, but not limited to, treating a wide range of states of HIV infection : AIDS, ARC (AIDS related complex), both symptomatic and asymtomatic, and actual or potential exposure to HIV. For example, the compounds of this invention are useful in preventing infection by HIV after suspected past exposure to HIV by, e.g., blood transfusion, accidental needle stick, or exposure to patient blood during surgery.

For these purposes, the peptides of the present invention may be administered orally, parenterally (including subcutaneous injections, intravenous, intramuscular, intrasternal injection or infusion techniques), by inhalation spray, or rectally, in dosage unit formulations containing conventional non-toxic, pharmaceutically-acceptable carriers, adjuvants and vehicles.

Thus, in accordance with the present invention there is further provided a method of treating and a pharmaceutical composition for treating renin-associated hypertension, hyperaldosteronism, and/or congestive heart failure. This treatment involves administering to a patient in need of such treatment a pharmaceutical composition comprising a pharmaceutical carrier and a therapeutically-effective amount of a peptide of the formula :

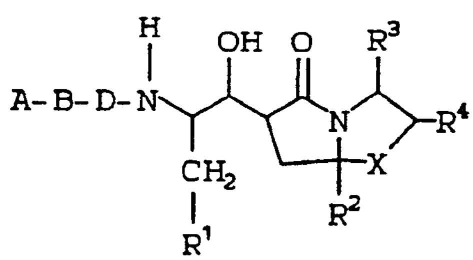

wherein A, B, D, $R^1$, $R^2$, $R^3$, $R^4$ and X are defined above, or a pharmaceutically-acceptable salt thereof.

In addition, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the presnet invention, or a pharmaceutically-acceptable salt thereof.

These pharmaceutical compositions may be in the form of orally-administrable suspensions or tablets ; nasal sprays ; sterile injectable preparations, for example, as sterile injectable aqueous or oleagenous suspensions ; or suppositories.

When administered orally as a suspension, these compositions may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

Dosage levels of the order of 0.02 to 2.0 grams-per-day are useful in the treatment of the above-indicated conditions, with oral doses two-to-five times higher. For example, renin- associated hypertension and hyperaldosteronism are effectively treated by the administration of from 10 to 50 milligrams of the compound per kilogram of body weight from one to three times per day. It will be understood, however, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the novel renin-inhibitory peptides of Formula I with one or more antihypertensive agents selected from the group consisting of diuretics, α- and/or β-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as :

Diuretics : acetazolamide ; amiloride ; bendroflumethiazide ; benzthiazide ; bumetanide ; chlorothiazide ; chlorthalidone ; cyclothiazide ; ethacrynic acid ; furosemide ; hydrochlorothiazide ; hydroflumethiazide ; indacrinone (racemic mixture, or as either the (+) or (-) enantiomer alone, or a manipulated ratio, e.g., 9 :1 of said enantiomers, respectively) ; metolazone ; methyclothiazide ; muzolimine ; polythiazide ; quinethazone ; sodium ethacrynate ; sodium nitroprusside ; spironolactone ; ticrynafen ; triamterene ; trichlormethiazide ;

α-Adrenergic Blocking Agents : dibenamine ; phentolamine ; phenoxybenzamine ; prazosin ; tolazoline ;

β-Adrenergic Blocking Agents : atenolol ; metoprolol ; nadolol ; propranolol ; timolol ;
((±)-2-[3-(tert-butylamino)-2-hydroxypropoxy]-2-furananilide) (ancarolol) ;
(2-acetyl-7-(2-hydroxy-3-isopropylaminopropoxy)benzofuran HCl) (befunolol) ;
((±)-1-(isopropylamino)-3-(p-(2-cyclopropylmethoxyethyl)-phenoxy)-2-propranol HCl) (betaxolol) ;
(1-[(3,4-dimethoxyphenethyl)amino]-3-(m-tolyloxy)-2-propanol HCl) (bevantolol) ;
((±)-1-(4-((2-isopropoxyethoxy)methyl)phenoxy)-3-isopropylamino-2-propanol)fumarate) (bisoprolol) ;
(4-(2-hydroxy-3-[4-(phenoxymethyl)-piperidino]-propoxy)-indole) ;
(carbazolyl-4-oxy-5,2-(2-methoxyphenoxy)-ethylamino-2-propanol) ;
(1-((1,1-dimethylethyl)amino)-3-((2-methyl-1H-indol-4-yl)oxy)-2-propanol benzoate) (bopindolol) ;
(1-(2-exobicyclo[2.2.1]-hept-2-ylphenoxy)-3-[(1-methyl-ethyl)-amino]-2-propanol HCl) (bornaprolol) ;
(o-[2-hydroxy-3-[(2-indol-3-yl-1,1-dimethylethyl)-amino]propoxy]benzonitrile HCl) (bucindolol) ;
(α-[(tert-butylamino)methyl]-7-ethyl-2-benzofuran-methanol) (bufuralol) ;
(3-[3-acetyl-4-[3-(tert-butylamino)-2-hydroxypropyl]-phenyl]-1,1-diethylurea HCl) (celiprolol) ;
((±)-2-[2-[3-[(1,1-dimethylethyl)amino]-2-hydroxypropoxy]phenoxy]-N-methylacetamide HCl) (cetamolol) ;
(2-benzimidazolyl-phenyl(2-isopropylaminopropanol)) ;
((±)-3'-acetyl-4'-(2-hydroxy-3-isopropylaminopropoxy)-acetanilide HCl)-(diacetolol) ;
(methyl-4-[2-hydroxy-3-[(1-methylethyl)aminopropoxy]]-benzenepropanoate HCl) (esmolol) ;
(erythro-DL-1-(7-methylindan-4-yloxy)-3-isopropyl-aminobutan-2-ol) ;
(1-(tert-butylamino)-3-[O-(2-propynyloxy)phenoxy]-2-propanol (pargolol) ;
(1-(tert-butylamino)-3-[o-(6-hydrazino-3-pyridazinyl)-phenoxy]-2-propanol diHCl) (prizidilol) ;
((-)-2-hydroxy-5-[(R)-1-hydroxy-2-[(R)-(1-methyl-3-phenylpropyl)amino]ethyl]benzamide) ;
(4-hydroxy-9-[2-hydroxy-3-(isopropylamino)-propoxy]-7-methyl-5H-furo[3,2-g][1]-benzopyran-5-one) (iprocrolol) ;
((-)-5-(tert-butylamino)-2-hydroxypropoxy]-3,4-dihydro-1-(2H)-naphthalenone HCl) (levobunolol) ;
(4-(2-hydroxy-3-isopropylamino-propoxy)-1,2-benzisothiazole HCl) ;
(4-[3-(tert-butylamino)-2-hydroxypropoxy]-N-methylisocarbostyril HCl) ;

((±)-N-2-[4-(2-hydroxy-3-isopropyl aminopropoxy)-phenyl]ethyl-N'-isopropylurea) (pafenolol) ;
(3-[[(2-trifluoroacetamido)ethyl]amino]-1-phenoxypropan-2-ol) ;
(N-(3-(o-chlorophenoxy)-2-hydroxypropyl)-N'-(4'-chloro-2,3-dihydro-3-oxo-5-pyridazinyl)ethyl-enediamine) ;
((±)-N-[3-acetyl-4-[2-hydroxy-3-[(1-methylethyl)-amino]-propoxy]phenyl]butanamide) (acebutolol) ;
((±)-4'-[3-(tert-butylamino)-2-hydroxypropoxy]spiro-[cyclohexane-1,2'-indan]-1'-one) (spirendolol) ;
(7-[3-[[2-hydroxy-3-[(2-methylindol-4-yl)oxy]propyl]-amino]butyl]thiophylline) (teoprolol) ;
((±)-1-tert-butylamino-3-(thiochroman-8-yloxy)-2-propanol) (tertatolol) ;
((±)-1-tert-butylamino-3-(2,3-xylyloxy)-2-propanol HCl) (xibenolol) ;
(8-[3-(tert-butylamino)-2-hydroxypropoxy]-5-methyl-coumarin) (bucumolol) ;
(2-(3-(tert-butylamino)-2-hydroxy-propoxy)benzonitrile HCl) (bunitrolol) ;
((±)-2'-[3-(tert-butylamino)-2-hydroxypropoxy-5'-fluorobutyrophenone) (butofilolol) ;
(1-(carbazol-4-yloxy)-3-(isopropylamino)-2-propanol) (carazolol) ;
(5-(3-tert-butylamino-2-hydroxy)propoxy-3,4-dihydro-carbostyril HCl) (carteolol) ;
(1-(tert-butylamino)-3-(2,5-dichlorophenoxy)-2-propanol) (cloranolol) ;
(1-(inden-4(or 7)-yloxy)-3-(isopropylamino)-2-propanol HCl) (indenolol) ;
(1-isopropylamino-3-[(2-methylindol-4-yl)oxy]-2-propanol) (mepindolol) ;
(1-(4-acetoxy-2,3,5-trimethylphenoxy)-3-isopropylamino-propan-2-ol) (metipranolol) ;
(1-(isopropylamino)-3-(o-methoxyphenoxy)-3-[(1-methylethyl)amino]-2-propanol) (moprolol) ;
((1-tert-butylamino)-3-[(5,6,7,8-tetrahydro-cis-6,7-dihydroxy-1-naphthyl)oxy]-2-propanol) (nadolol) ;
((S)-1-(2-cyclopentylphenoxy)-3-[(1,1-dimethylethyl)-amino]-2-propanol sulfate (2 :1)) (penbutolol) ;
(4'-[1-hydroxy-2-(amino)ethyl]methanesulfonanilide) (sotalol) ;
(2-methyl-3-[4-(2-hydroxy-3-tert-butylaminopropoxy)-phenyl]-7-methoxy-isoquinolin-1-(2H)-one) ;
(1-(4-(2-(4-fluorophenyloxy)ethoxy)phenoxy)-3-iso-propylamino-2-propanol HCl) ;
((-)-p-[3-[(3,4-dimethoxyphenethyl)amino]-2-hydroxypropoxy]-β-methylcinnamonitrile) (pacrinolol) ;
((±)-2-(3'-tert.butylamino-2'-hydroxypropylthio)-4-(5'-carbamoyl-2'-thienyl)thiazole HCl) (arotinolol) ;
((±)-1-[p-[2-(cyclopropylmethoxy)ethoxy]phenoxy]-3-(isopropylamino)-2-propanol) (cicloprolol) ;
((±)-1-[(3-chloro-2-methylindol-4-yl)oxy]-3-[(2-phenoxyethyl)amino]-2-propanol) (indopanolol) ;
((±)-6-[[2-[[3-(p-butoxyphenoxy)-2-hydroxypropyl]-amino]ethyl]amino]-1,3-dimethyluracil) (pirepolol) ;
(4-(cyclohexylamino)-1-(1-naphtholenyloxy)-2-butanol) ;
(1-phenyl-3-[2-[3-(2-cyanophenoxy)-2-hydroxypropyl]-aminoethyl]hydantoin HCl) ;
(3,4-dihydro-8-(2-hydroxy-3-isopropylaminopropoxy)-3-nitroxy-2H-1-benzopyran) (nipradolol) ;
<u>α- and β-Adrenergic Blocking Agents</u> :
((±)-1-tert-butylamino)-3-[o-[2-(3-methyl-5-isoxazolyl)vinyl]phenoxy]-2-propanol) (isoxaprolol) ;
(1-isopropylamino-3-(4-(2-nitroxyethoxy)phenoxy)-2-propanol HCl) ;
(4-hydroxy-α-[[3-(4-methoxyphenyl)-1-methylpropyl]-aminomethyl]-3-(methylsulfinyl)-benzmethanol HCl) (sulfinalol) ;
(5-[1-hydroxy-2-[[2-(o-methoxyphenoxy)ethyl]amino]-ethyl]-2-methylbenzenesulfonamide HCl) ;
(5-[1-hydroxy-2-[(1-methyl-3-phenylpropyl)amino]-ethyl]-salicylamide HCl) (labetalol) ;
(1-((3-chloro-2-methyl-1H-indol-4-yl)oxy)-3-((2-phenoxyethyl)amino)-2-propanol-hydrogenmalonate) (ifendolol) ;
(4-(2-hydroxy-3-[(1-methyl-3-phenylpropyl)amino]-propoxy)benzeneacetamide) ;
(1-[3-[[3-(1-naphthoxy)-2-hydroxypropyl]-amino]-3,3-dimethyl-propyl]-2-benzimidazolinone) ;
(3-(1-(2-hydroxy-2-(4-chlorophenylethyl)-4-piperidyl)-3,4-dihydroxy)quinoxolin-2(1H)-one) ;
<u>CNS-Acting Agents</u> : clonidine ; methyldopa ;
<u>Adrenergic Neuron Blocking Agents</u> : guanethidine ; reserpine and other rauwolfia alkaloids such as rescinnamine ;
<u>Vasodilators</u> : diazoxide ; hydralazine ; minoxidil ;
<u>Angiotensin I Converting Enzyme Inhibitors</u> :
1-(3-mercapto-2-methyl-1-oxopropyl)-L-proline (captopril) ;
(1-(4-ethoxycarbonyl-2,4(R,R)-dimethylbutanoyl)-indoline-2(S)-carboxylic acid) ;
(2-[2-[[1-(ethoxycarbonyl)-3-phenyl-propyl]amino]-1-oxopropyl]-1,2,3,4-tetrahydro-3-isoquinoline carboxylic acid) ;
((S)-1-[2-[[1-(ethoxycarbonyl)-3-phenylpropyl]amino]-1-oxopropyl]octahydro-1H-indole-2-carboxylic acid HCl) ;
(N-cyclopentyl-N-(3-(2,2-dimethyl-1-oxopropyl)thiol-2-methyl-1-oxopropyl)glycine) (pivalopril) ;
((2R,4R)-2-(2-hydroxyphenyl)-3-(3-mercaptopropionyl)-4-thiazolidinecarboxylic acid) ;
(1-(N-[1(S)-ethoxycarbonyl-3-phenylpropyl]-(S)-alanyl)-cis,syn-octahydroindol-2(S)-carboxylic acid HCl) ;

((-)-(S)-1-[(S)-3-mercapto-2-methyl-1-oxopropyl]-indoline-2-carboxylic acid) ;
([1(S),4S]-1-[3-(benzoylthio)-2-methyl-1-oxopropyl]-4-phenylthio-L-proline ;
(3-([1-ethoxycarbonyl-3-phenyl-(1S)-propyl]amino)-2,3,4,5-tetrahydro-2-oxo-1-(3S)-benzazepine-1-acetic acid HCl) ;
(N-(2-benzyl-3-mercaptopropanoyl)-S-ethyl-L-cysteine) and the S-methyl analogue ;
(N-(1(S)-ethoxycarbonyl-3-phenylpropyl)-L-alanyl-L-proline maleate) (enalapril) ;
N-[1-(S)-carboxy-3-phenylpropyl]-L-alanyl-1-proline ;
$N^2$-[1-(S)-carboxy-3-phenylpropyl]-L-lysyl-L-proline (lisinopril) ;
Calcium Channel Blockers :
α-[3-[[2-(3,4-dimethoxyphenyl)ethyl]methylamino]-propyl]-3,4-dimethoxy-α-(1-methylethyl)benzene-acetonitrile (verapamil) ;
1,4-dihydro-2,6-dimethyl-4-(2-nitrophenyl)-3,5-pyridinedicarboxylic acid dimethyl ester (nifedipine) ;
2-(2,2-dicyclohexylethyl)piperidine (perhexiline) ;
N-(1-methyl-2-phenylethyl)-phenylbenzenepropanamine (prenylamine) ;
3-(aminosulfonyl)-4-chloro-N-(2,3-dihydro-2-methyl-1H-indol-1-yl)benzamide (indapamide) ;
(2'-(2-diethylaminoethoxy)-3-phenylpropiophenone (etafenone) ;
(4-[4,4-bis-(4-fluorophenyl)butyl]-N-(2,6-dimethylphenyl)-1-piperazineacetamide) (lidoflazine) ;
(2-(N-benzyl-N-methylamino)ethylmethyl-2,6-dimethyl-4-(m-nitrophenyl)-1,4-dihydro-3,5-pyridinedicarboxylate HCl) (nicardipine) ;
(N-(3,4-dimethoxyphenethyl)-2-(3,4-dimethoxyphenyl)-N-methyl-m-dithiane-2-propylamine-1,1,3,3-tetra oxide) (tiapamil) ;
(5,6-dimethoxy-2-(3-[(α-(3,4-dimethoxy)phenylethyl)-methylamino]propyl)phthalimidine) (falipamil) ;
(β-[(2-methylpropoxy)methyl]-N-phenyl-N-phenylmethyl-1-pyrrolidineethanamine HCl monohydrate) (bepridil) ;
((+)-cis-3-(acetyloxy)-5-[2-(dimethylamino)ethyl]-2,3-dihydro-2-(4-methoxyphenyl)-1,5-benzothiazepin-4-(5H)-one) (diltiazem) ;
((E)-1-[bis-(p-fluorophenyl)methyl]-4-cinnamylpiperazine di HCl) (flunarizine) ;
(5-[(3,4-dimethoxyphenethyl)methylamino]-2-isopropyl-2-(3,4,5-trimethoxyphenyl)valeronitrile (gallopamil) ;
(ethylmethyl(2,3-dichlorophenyl)-1,4-dihydro-2,6-dimethyl-3,5-pyridinedicarboxylate (felodipine) ;
(isopropyl-2-methoxyethyl-1,4-dihydro-2,6-dimethyl-4-(3-nitrophenyl)-3,5-pyridinecarboxylate) (nimodipine) ;
(3-ethyl-5-methyl-1,4-dihydro-2,6-dimethyl-4-(3-nitrophenyl)-3,5-pyridine-dicarboxylate) (nitrendipine) ;

Other Antihypertensive Agents : aminophylline ; cryptenamine acetates and tannates ; deserpidine ; meremethoxylline procaine ; pargyline ; trimethaphan camsylate ; and the like, as well as admixtures and combinations thereof.

Typically, the individual daily dosages for these combinations can range from about one-fifth of the minimally-recommended clinical dosages to the maximum recommended levels for the entities when they are given alone. Coadministration is most readily accomplished by combining the active ingredients into a suitable unit dosage form containing the proper dosages of each. Other methods of coadministration are, of course, possible.

The renin-inhibitory novel peptides of the present invention may also be utilized in in vivo or in vitro diagnostic methods for the purpose of establishing the significance of renin as a causative or contributory factor in hypertension, hyperaldosteronism or congestive heart failure in a particular patient.

In the in vivo method, a novel peptide of the present invention is administered to a patient, preferably by intravenous injection, although parenteral administration is also suitable, at a hypotensive dosage level in a single dose of from 0.1 to 10 mg per kg of body weight, and the resulting transitory fall in blood pressure, if it occurs, indicates supranormal plasma renin levels.

In vitro methods which may be employed involve incubating a body fluid, preferably plasma, with a novel peptide of the present invention according to methods described in Boger et al., J. Med. Chem., 1985, 28, 1779-1790.

The following are intended to exemplify the present invention, without, however, limiting it.

## EXAMPLE 1

### Synthesis of starting lactams

A. Preparation of 5(S)-Allyl-7-Oxa-9(S)-Indolizidin- 3-One

Step 1 : 5(S)-Allyloxymethyl-2-Pyrrolidinone and 1-Allyl-5(S)-Hydroxymethyl-2-Pyrrolidinone

To a mechanically stirred, 0°C solution of 5(S)-hydroxymethyl-2-pyrrolidinone (40.0 g ; 0.348 mol ; prepared by the method of Chem. Pharm. Bull. Japan (1980), Saijo et al., 28, 1449, in 2 L of dry THF under an atmosphere of nitrogen, was added n-butyllithium (155 mL of a 2.5 molar solution in hexanes ; 0.384 mol) dropwise over a period of 2 hours. The resulting suspension was stirred at ambient temperature for 3 hours, at which time allyl bromide (48.0 g ; 0.397 mol) was added dropwise over a period of 1 hour. The reaction mixture was stirred at ambient temperature for 16 hours, filtered through Celite, and the filtrate solvents were removed under reduced pressure. The residue was flash chromatographed over silica gel using a gradient elution, 2% $MeOH/CH_2Cl_2$ to 8% $MeOH/CH_2Cl_2$, giving in order of elution from the column, 1-allyl-5(S)- allyloxymethyl-2-pyrrolidinone (19.0 g ; 28%), 1-allyl-5(S)-hydroxymethyl-2-pyrrolidinone (9.71 g ; 18%) and 5(S)-allyloxymethyl-2-pyrrolidinone (12.4 g ; 23%).

TLC (Silica Gel 60-F254, 5% $MeOH/CH_2Cl_2$) $R_f$=0.58 ;

'H NMR (300 MH$_z$, $CD_3OD$) δ = 1.86 (M, 1H), 2.15-2.43 (M, 3H), 3.38 (dd, 1H), 3.46 (dd, 1H), 3.82 (M, 1H), 4.01 (dm, 2H), 5.16 (dm, 1H), 5.27 (dm, 1H), 5.91 (s, 1H).

Step 2 : 5(S)-Acetoxy-7-Oxa-9(S)-Indolizidin-3-One

Into a -78°C solution of 5(S)-allyloxymethyl-2-pyrrolidinone (5.0 g ; 32.3 mmol) in 10% $MeOH/CH_2Cl_2$ (100 mL) was bubbled a stream of ozone until a pale blue color persisted (ca. 15 min). Excess ozone was purged by bubbling nitrogen through the solution until a colorless solution was obtained. Dimethyl sulfide (3 mL) was added to the solution, which was then allowed to warm to ambient temperature for 2 hours. The solvents were removed under reduced pressure, the residue was dissolved in dimethyl sulfide (8 mL), and the resulting solution was refluxed for 4 hours.

Excess dimethyl sulfide was removed under reduced pressure, and the resulting oil was stored at 35°C in vacuo (0.2 torr) for 16 hours to remove DMSO.

Isomer I : TLC (Silica Gel 60-F254, 5% $MeOH/CH_2Cl_2$) $R_f$=0.65 ;

'H NMR (300 MHz, $CD_3OD$) δ = 1.58 (M, 1H), 2.10 (M.1H), 2.38 (M, 2H), 3.22 (dd, 1H), 3.31 (t, 1H), 3.75 (M, 1H), 3.87 (dd, 1H), 3.98 (dd, 1H), 5.11 (dd, 1H).

Isomer II : TLC (Silica Gel 60-F254, 5% $MeOH/CH_2Cl_2$) $R_f$=0.27 ;

'H NMR (300 MHz, $CD_3OD$) δ = 1.55 (M, 1H), 2.14 (M,1H), 2.3-2.5 (M, 2H), 3.15 (t, 1H), 3.45 (dd, 1H), 3.89 (d, 1H), 4.0-4.1 (M, 2H), 5.30 (d, 1H).

The crude ozonolysis product was dissolved in 1 :1 acetic anhydride/pyridine (10 mL), DMAP (0.10 g ; 0.82 mmol) was added, and the resulting solution was stored at ambient temperature for 6 hours, and then at 5°C for 18 hours. Excess acetic anhydride and pyridine were removed under reduced pressure and the residue was flash chromatographed over silica gel using a gradient elution of 15% to 35% ether/$CH_2Cl_2$ to give the title compound (4.4 g ; 68%) as a colorless oil.

TLC (Silica Gel 60-F254, 40% EtOAC/$CH_2Cl_2$, 2 developments) $R_f$=0.59 ;

'H NMR (300 MHz $CDCl_3$) δ = 1.58 (M, 1H), 2.18 (M, 1H), 2.09 (S, 3H), 2.43 (M, 2H), 3.18 (t, 1H), 3.52 (dd, 1H), 4.00 (M, 1H), 4.09 (d, 1H), 4.11 (dd, 1H), 6.35 (d, 1H).

Step 3 : 5(S)-Allyl-7-Oxa-9(S)-Indolizidin-3-One

To a 0°C solution of 5(S)-acetoxy-7-oxa-9(S)-indolizidin-3-one (4.00 g ; 20.1 mmol) in dry $CH_2Cl_2$ (20 mL) under an atmosphere of nitrogen was added allyltrimethylsilane (6.8 g ; 60 mmol) followed by borontrifluoride etherate (2.7 ml ; 22.1 mmol). The solution was stirred at 0°C for 1 hour, and then at ambient temperature for 1 hour. The reaction was quenched with saturated aqueous $NaHCO_3$ (50 mL), the layers were separated, and the aqueous phase was extracted with $CH_2Cl_2$ (3 x 50 mL). The combined organic phases were dried ($MgSO_4$), filtered, and the solvents were removed under reduced pressure. The residue was flash chromatographed over silica gel using a gradient elution of 5% to 25% ether/$CH_2Cl_2$ to afford the title compound as a colorless oil (3.50 g ; 96%).

TLC (Silica Gel 60-F254, 40% diethyl ether/$CH_2Cl_2$, 2 developments) $R_f$=0.48 ;

'H NMR (300 MHz, $CDCl_3$) δ = 1.50 (M, 1H), 2.11 (M, 1H), 2.35-2.55 (M, 4H), 3.07 (t, 1H), 3.42 (dd, 1H), 3.81 (M, 1H), 3.82 (d, 1H), 4.01 (dd, 1H), 4.06 (td, 1H), 5.08 (dm, 1H), 5.12 (dm, 1H), 5.78 (ddt, 1H).

B. Preparation of 5(S)-(2-Methylallyl)-7-Oxa-9(S)-Indolizidin-3-One

The title compound was prepared from 5(S)-acetoxy-7-oxa-9(S)-indolizidin-3-one (1.12 g ; 5.63 mmol),

methallyltrimethylsilane (2.16 g ; 16.9 mmol), and borontrifluoride etherate (0.76 mL ; 6.2 mmol) using the conditions described in Example 1A, Step 3. The crude product was purified by crystallization from 10 :1 hexane-ether (0.99 g ; 90% yield).

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=5.29 min.

'H NMR (300 MHz, $CDCl_3$) $\delta$ = 1.48 (M, 1H), 1.79 (s, 3H), 2.08 (M, 1H), 2.3-2.5 (M, 4H), 3.41 (dd, 1H), 3.79 (d, 1H), 3.82 (M, 1H), 3.98 (dd, 1H), 4.17 (td, 1H), 4.75 (M, 1H), 4.80 (M, 1H).

IR ($CDCl_3$) 2960, 2860, 1680, 1460, 1440, 1425, 1385, 1365, 1310, 1265, 1110, $cm^{-1}$.

C. Preparation of 5(S)-(2-Methylpropyl)-7-Oxa-9(S)-Indolizidin-3-One

The title compound is prepared catalytic hydrogenation of 5(S)-(2-methylallyl-7-oxa-9(S)-indolizidin-3-one using 10% Pd on carbon in EtOAc. The catalyst is removed by filtration, the filtrate solvent is removed under reduced pressure, and the residue is purified by chromatography on silica gel to give 5(S)-(1-methyl-ethyl)-7-oxa-9(S)-indolizidin-3-one.

D. Preparation of 5(S)-Cyclopentyl-7-Oxa-9(S)-Indolizidin-3-One

5(S)-Acetoxy-7-oxa-9(S)-indolizidin-3-one (785 mg ; 3.94 mmol) was treated with 3-trimethyl silylcyclopentene (1.65 g ; 11.8 mmol) and borontrifluoride etherate (0.53 mL ; 4.3 mmol) using the procedure described in Example 1A, Step 3. The product was purified by flash column chromatography (silica gel, gradient elution using 5% to 25% ether/$CH_2Cl_2$).

Partial 'H NMR (300 MHz, $CDCl_3$) major isomer : $\delta$ = 5.62, 5.82 ; minor isomer : $\delta$ = 5.51, 5.75

The mixture of isomers was hydrogenated using 10% Pd on carbon (0.10 g) in EtOAc solution (10 mL) under 1 atmosphere of $H_2$ for 6 hours. The catalyst was removed by filtration, the filtrate solvent was removed under reduced pressure, and the residue was purified by passage through a short column of silica gel (10% ether in $CH_2Cl_2$) to give the title compound as a colorless oil (708 mg ; 86% yield over two steps).

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=6.08 min.

'H NMR (300 MHz, $CDCl_3$) $\delta$ = 1.1-1.8 (m, 9H), 2.07 (m, 1H), 2.25-2.50 (m, 3H), 2.99 (t, 1H), 3.85 (dd, 1H), 3.66 (dd, 1H), 3.79 (d, 1H), 3.80 (m, 1H), 3.91 (dd, 1H).

IR($CDCl_3$) 2950, 2860, 1670, 1450, 1430, 1415, 1375, 1355, 1300, 1255, 1105 $cm^{-1}$.

E. Preparation of 5(S)-Propyl-7-Oxa-9(S)-Indolizidin-3-One

The title compound is prepared catalytic hydrogenation of 5(S)-allyl-7-oxa-9(S)-indolizidin-3-one using 10% Pd on carbon in EtOAc. The catalyst is removed by filtration, the filtrate solvent is removed under reduced pressure, and the residue is purified by chromatography on silica gel to give 5(S)-propyl-7-oxa-9(S)-indolizidin-3-one.

F. Preparation of 7-Oxa-9(S)-Indolizidin-3-One

The title compound is prepared form 5(S)-acetoxy-7-oxa-9(S)-indolizidin-3-one, triethylsilane, and borontrifluoride etherate using the conditions described in Example 1A, Step 3.

G. Preparation of 5(S)-Vinyl-7-Oxa-9(S)-Indolizidin-3-One

The title compound is prepared from 5(S)-acetoxy-7-oxa-9(S)-indolizidin-3-one, trimethylvinylsilane, and borontrifluoride etherate using the conditions described in Example 1A, Step 3.

H. Preparation of 5(S)-Ethyl-7-Oxa-9(S)-Indolizidin-3-One

The title compound is prepared catalytic hydrogenation of 5(S)-vinyl-7-oxa-9(S)-indolizidin-3-one using 10% Pd on carbon in EtOAc. The catalyst is removed by filtration, the filtrate solvent is removed under reduced pressure, and the residue is purified by chromatography on silica gel to give 5(S)-ethyl-7-oxa-9(S)-indolizidin-3-one.

I. Preparation of 5(R)-(2-Thieno)-7-Oxa-9(S)-Indolizidin-3-One

The title compound is prepared from 5(S)-acetoxy-7-oxa-9(S)-indolizidin-3-one, 2-thieno trimethylsilane, and borontrifluoride etherate using the conditions described in Example 1A, Step 3.

J. Preparation of 5(R)-(2-Furyl)-7-Oxa-9(S)-Indolizidin-3-One

The title compound is prepared from 5(S)-acetoxy-7-oxa-9(S)-indolizidin-3-one, 2-furyl trimethylsilane, and borontrifluoride etherate using the conditions described in Example 1A, Step 3.

K. Preparation of 5(S)-Phenyl-7-Oxa-9(S)-Indolizidin-3-One

The title compound is prepared from 5(S)-acetoxy-7-oxa-9(S)-indolizidin-3-one, phenyl trimethylsilane, and borontrifluoride etherate using the conditions described in Example 1A, Step 3.

L. Preparation of 5(S)-Cyano-7-Oxa-9(S)-Indolizidin-3-One

The title compound is prepared from 5(S)-acetoxy-7-oxa-9(S)-indolizidin-3-one, cyano trimethylsilane, and borontrifluoride etherate using the conditions described in Example 1A, Step 3.

N. Preparation of 5(R)-Methoxycarbonyl-7-Oxa-9(S)-Indolizidin-3-One

The title compound is prepared by ozonolysis of a solution of 5(S)-vinyl-7-oxa-9(S)-indolizidin-3-one in methanol/dichloromethane at -78°C, followed by a work-up utilizing hydrogen peroxide and esterification by treatment with diazomethane.

P. Preparation of 5(S)-(2-Hydroxyethyl)-7-Oxa-9(S)-Indolizidin-3-One

The title compound is prepared by ozonolysis of a solution of 5(S)-allyl-7-oxa-9(S)-indolizidin-3-one in methanol/dichloromethane at -78°C, followed by a reductive work-up utilizing sodium borohydride in methanol.

Q. Preparation of 5(S)-[2-(2-Propylaminocarbamoyl)-hydroxyethyl]-7-Oxa-9(S)-Indolizidin-3-One

The title compound is prepared by reaction of 5(S)-(2-hydroxyethyl)-7-oxa-9(S)-indolizidin-3-one with isopropyl isocyanate in the presence of triethylamine.

R. Preparation of 5(R)-Carboxamide-7-Oxa-9(S)-Indolizidin-3-One

The title compound is prepared from 5(S)-cyano-7-oxa-9(S)-indolizidin-3-one by hydrolysis with an alkaline solution of hydrogen peroxide.

S. Preparation of 5(S)-(2-N,N-Diethylaminoethyl)-7-Oxa-9(S)-Indolizidin-3-One

The title compound is prepared by ozonolysis of a solution of 5(S)-allyl-7-oxa-9(S)-indolizidin-3-one in methanol/dichloromethane at -78°C, followed by a reductive work-up with dimethylsulfide, to give the aldehyde derivative, which is reductively aminated using diethylamine hydrochloride and $NaBH_4$ to give 5(S)-(2-diethylaminoethyl)-7-oxa-9(S)-indolizidin-3-one.

T. Preparation of 9-Methyl-7-Oxa-Indolizidin-3-One

Condensation of methyl-2-nitropropionate with methyl acrylate in the presence of triethylamine gives dimethyl 2-methyl-2-nitroglutarate. Reduction with Raney nickel under an atmosphere of hydrogen gives a 5-methyl-5-methoxycarbonyl-2-pyrrolidinone. Alkylation of the amide nitrogen is accomplished by treatment with one equivalent of sodium hydride followed by addition of allyl bromide. The resultant product is then reacted with two equivalents of diisobutylaluminum hydride to give 1-allyl-5-hydroxymethyl-5-methyl-2-pyrrolidinone. This is then reacted with ozone employing a reductive workup utilizing sodium borohydride. The resultant diol is then reacted with tosyl chloride followed by sodium hydride to give 9-methyl-7-oxa-indolizidin-3- one

U. Preparation of 9-Ethyl-7-Oxa-Indolizidin-3-One

The title compound is prepared from methyl-2-nitrobutyrate utilizing the methodology described in Example 1T.

V. Preparation of 6-Allyl-7-Oxa-9(S)-Indolizidin-3-One

Step 1 : 6-Acetoxy-7-Oxa-9(S)-Indolizidin-3-One

The title compound is prepared from 1-allyl-5(S)-hydroxymethyl-2-pyrrolidinone utilizing the procedures of Example 1A, Step 2.

Step 2 : 6-Allyl-7-Oxa-9(S)-Indolizidin-3-One

The title compound is prepared from 6(S)-acetoxy-7-oxa-9(S)-indolizidin-3-one, allyltrimethylsilane, and borontrifluoride etherate using the conditions described in Example 1A, Step 3.

W. Preparation of 6-Phenyl-7-Oxa-9(S)-Indolizidin-3-One

The title compound is prepared from 6(S)-acetoxy-7-oxa-9(S)-indolizidin-3-one, phenyltrimethylsilane, and borontrifluoride etherate using the conditions described in Example 1A, Step 3.

X. Preparation of 6-Vinyl-7-Oxa-9(S)-Indolizidin-3-One

The title compound is prepared from 6(S)-acetoxy-7-oxa-9(S)-indolizidin-3-one, vinyltrimethylsilane, and borontrifluoride etherate using the conditions described in Example 1A, Step 3.

Y. Preparation of 6-Carboxy-7-Oxa-9(S)-Indolizidin-3-One

The title compound is prepared by ozonolysis of a solution of 6(S)-vinyl-7-oxa-9(S)-indolizidin-3-one in methanol/dichloromethane at -78°C, followed by a work-up utilizing hydrogen peroxide.

Z. Preparation of 6-[(1-methylpiperidin-3-yl)aminocarbonyl]-7-Oxa-9(S)-Indolizidin-3-One

The title compound is prepared by coupling 6-carboxy-7-oxa-9(S)-indolizidin-3-one with 3-amino-1-methylpiperidine using BOP in DMF with the solution being kept at pH 8 by addition of DIPEA.

AA. Preparation of 6-(N,N-Diethylaminomethyl)-7-Oxa-9(S)-Indolizidin-3-One

Ozonolysis of 6(S)-allyl-7-oxa-9(S)-indolizidin-3-one with a neutral workup employing dimethyl sulfide gives the corresponding aldehyde. The title compound is obtained by reductive amination of the aldehyde using diethylamine hydrochloride and $NaCNBH_3$.

BB. Preparation of 6-Allyl-9-Methyl-7-Oxa-Indolizidin-3-One

The title compound is prepared by first reacting 1-allyl-5-hydroxymethyl-5-methyl-2-pyrrolidinone with ozone employing a neutral workup with dimethyl sulfide to give 6-hydroxy-9-methyl-7-oxa-indolizidin-3-one. As described in Example 1A, Step 3, the alcohol is acetylated with acetic anhydride in the presence of a pyridine base, converted to an acyl imminium species under the influence of boron trifluoride etherate and trapped *in situ* with allyltrimethylsilane to give the desired product, 5-allyl-9-methyl-7-oxa-indolizidin-3-one

CC. Preparation of 5(S)-Allyl-6-Methyl-7-Oxa-Indolizidin-3-One

Step 1 : Preparation of 5(S)-[1-(Methoxycarbonyl)-ethoxymethyl]-2-Pyrrolidinone

Alkylation of 5(S)-hydroxymethyl-2-pyrrolidinone is accomplished by deprotonation using a base such as sodium hydride, potassium hydride or butyl lithium, followed by the addition of methyl 2-bromopropionate to give the title compound.

Step 2 : Preparation of 5(S)-Acetoxy-6(S)-Methyl-7-Oxa-Indolizidin-3-One

Reduction of the ester group in 5(S)-[1-(methoxycarbonyl)ethoxymethyl]-2-pyrrolidinone with diisopropylaluminum hydride gives the aldehyde which cyclizes in situ to give 5-hydroxy-6-methyl-7-oxa-9(S)-indolizidin-3-one. Reaction of the alcohol with acetic anhydride in the presence of a pyridine base gives 5(S)-acetoxy-6-methyl-7-oxa-9(S)-indolizidin-3-one.

Step 3 : Preparation of 5(S)-Allyl-6-Methyl-7-Oxa-9(S)-Indolizidin-3-One

5(S)-Acetoxy-6-methyl-7-oxa-9(S)-indolizidin-3-one is reacted with allyltrimethylsilane in the presence of borontrifluoride etherate using the conditions described in Example 1A, Step 3 to give 5(S)-allyl-6)-methyl-7-oxa-9(S)-indolizidin-3-one.

DD. Preparation of 8-Methyl-7-Oxa-9(S)-Indolizidin-3-One

Step 1 : Preparation of 1-Allyl-5(S)-(1-Hydroxyethyl)-2-Pyrrolidinone

Oxidation of 1-allyl-5(S)-hydroxymethyl-2-pyrrolidinone to the corresponding aldehyde is accomplished with pyridinium chlorochromate. Addition of the Grignard reagent derived from methyl bromide followed by aqueous work-up gives 1-allyl-5(S)-(1-hydroxyethyl)-2-pyrrolidinone.

Step 2 : Preparation of 1-(2-hydroxyethyl)-5(S)-(1-Hydroxethyl)-2-Pyrrolidinone

Ozonolysis of 1-allyl-5(S)-(1-hydroxyethyl)-2-pyrrolidinone employing a basic workup with sodium borohydride gives the desired diol, 1-(2-hydroxyethyl)-5(S)-(1-hydroxyethyl)-2-pyrrolidinone.

Step 3 : Preparation of 8(S)-Methyl-7-Oxa-9(S)-Indolizidin-3-One

Treatment of the diol with tosyl chloride and then a suitable base, e.g., sodium hydride in THF, results in cyclization to the desired product, 8-methyl-7-oxa-9(S)-indolizidin-3-one.

EE. Preparation of 5(S)-Allyl-8-Methyl-7-Oxa-9(S)-Indolizidin-3-One

Step 1 : Preparation of 5(S)-(1-Hydroxyethyl)-2-Pyrrolidinone

Oxidation of 5(S)-hydroxymethyl-2-pyrrolidinone to the corresponding aldehyde with pyridinium chlorochromate followed by the addition of the Grignard reagent derived from methyl bromide gives 5(S)-(1-hydroxyethyl)-2-pyrrolidinone.

Step 2 : Preparation of 5(S)-Acetoxy-8(S)-Methyl-7-Oxa-9(S)-Indolizidin-3-One

5(S)-(1-Hydroxyethyl)-2-pyrrolidinone is alkylated by treatment with a suitable base, e.g., sodium hydride in THF, followed by addition of allyl bromide. Ozonolysis of 5(S)-(1-allyloxyethyl)-2-pyrrolidinone with a neutral workup employing dimethylsulfide followed by acetylation with acetic anhydride and a pyridine base gives 5(S)-acetoxy-8-methyl-7-oxa-9(S)-indolizidin-3-one.

Step 3 : Preparation of 5(S)-Allyl-8-Methyl-7-Oxa-9(S)-Indolizidin-3-One

5(S)-Acetoxy-8-methyl-7-oxa-9(S)-indolizidin-3-one is reacted with allyltrimethylsilane in the presence of borontrifluoride etherate using the conditions described in Example 1A, Step 3 to give 5(S)-allyl-8-methyl-7-oxa-9(S)-indolizidin-3-one.

FF. Preparation of 5(S)-Propyl-7-Hydroxy-9(S)-Indolizidin-3-One

The title compound is prepared from 4(S)-amino-1-heptene using the procedure of Hart, et. al., J. Am. Chem. Soc., 1980, 102, 397. Accordingly, 4(S)-amino-1-heptene (obtained from L-norvaline) is acylated by heating neat with succinic anhydride to give the succinimide derivative, which is reduced with $NaBH_4$ in methanol to the hydroxy-succinimide. Treatment with formic acid effects cyclization to give 5(S)-propyl-7-formyloxy-9(S)-indolizidin-3-one, hydrolysis of which with ($Na_2CO_3$ in MeOH) gives 5(S)-propyl-7-hydroxy-9(S)-indolizidin-3-one.

GG. Preparation of 5(S)-Propyl-6,7-Dehydro-9(S)-Indolizidin-3-One and 5(S)-Propyl-7,8-Dehydro-9(S)-Indolizidin-3-One

5(S)-Propyl-7-methanesulfonyl-9(S)-indolizidin-3-one is prepared by treatment of 5(S)-propyl-7-hydroxy-9(S)-indolizidin-3-one with methanesulfonyl chloride in the presence of pyridine. The sulfonyl ester is eliminated under basic conditions to give the corresponding dehydro-derivatives, 5(S)-propyl-6,7-dehydro-9(S)-indolizidin-3-one and 5(S)-propyl-7,8-dehydro-9(S)-indolizidin-3-one.

HH. Preparation of 5(S)-Propyl-6-Oxa-8(S)-(Pyrrolizidine)-3-One

Condensation of 5(S)-hydroxymethyl-2-pyrrolidinone with butyraldehyde by heating with toluenesulfonic acid in benzene with azeotropic remocal of water gives the desired 5(S)-propyl-6-oxa-8(S)-(pyrrolizidine)-3-one as described by Thottathil, et. al., J. Org. Chem., 1986, 51, 3140.

EXAMPLE 2

Synthesis of Boc-(ACHP)-lactams

A. Preparation of 5(S)-Allyl-2(S)-[2(S)-tert-Butyloxy-carbonylamino-3-Cyclohexyl-1(S)-Hydroxypropyl]-7-Oxo-9(S)-Indolizidin-3-One

To a 0°C solution of diisopropylamine (2.8 mL ; 20 mmol) in dry THF (40 mL) under an atmosphere of nitrogen was added n-butyllithium (11.4 mL of a 1.6 molar solution in hexanes ; 18.2 mmol). After being stirred for 10 min, the solution was cooled to -78°C and to it was added a solution of 5(S)-allyl-7-oxa-9(S)-indolizidin-3-one (2.75 g ; 15.2 mmol) in dry THF (5 mL) over a period of 10 min. The resulting solution was stirred at -78°C for 2.5 hours, at which time a -78°C solution of Boc-L-cyclohexylalaninal (4.26 g ; 16.7 mmol ; prepared by the method of Boger, et al., J. Med. Chem. (1985), 28, 1779) in dry THF (10 mL) was rapidly added via cannula. The reaction mixture was quenched after 3 min by the addition of aqueous ammonium chloride (25 mL). Ether (75 mL) was added and the organic phase was washed with 5% aqueous HCl (50 mL), water (50 mL), and saturated aqueous $NaHCO_3$ (100 mL). The organic layer was dried ($MgSO_4$), filtered, and the solvents were removed under reduced pressure. The four diastereomeric aldol products were separated by flash chromatography over silica gel using a gradient elution of 10% to 50% ether/$CH_2Cl_2$. The title compound was obtained as a colorless oil (1.32 g ; (20%).

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=9.95 min.

Partial 'H NMR (300 MHz, $CD_3OD$) $\delta$ = 1.43 (S, 9H), 2.46 (M, 2H), 2.63 (M, 1H), 3.08 (t, 1H), 3.40 (dd, 1H), 3.71 (M, 1H), 3.79 (d, 1H), 3.8-4.0 (M, 3H), 4.00 (dd, 1H), 5.05 (dm, 1H), 5.12 (dm, 1H), 5.79 (dddd, 1H).

The following compounds were obtained using the procedure given for the preparation of 5(S)-allyl-2(S)-[2(S)-tert-butyloxycarbonylamino-3-cyclohexyl-1(S)-hydroxypropyl]-7-oxo-9(S)-indolizidin-3-one, above, and exhibited NMR spectra which support the assigned structure :

B. 2(S)-Boc-(ACHP)-5(S)-Methallyl-7-Oxa-9(S)-Indolizidin-3-One

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=10.21 min.

Partial 'H NMR (300 MHz, $CD_3OD$) $\delta$ = 1.43 (s, 9H), 1.78 (s, 3H), 2.42 (m, 2H), 2.62 (ddd, 1H), 3.08 (t, 1H), 3.40 (dd, 1H) 3.77 (d, 1H), 3.80-3.95 (m, 4H), 4.12 (dt, 1H), 4.76 (m, 1H), 4.80 (m, 1H).

C. 2(S)-Boc-(ACHP)-5(S)-(2-Methylpropyl)-7-Oxa-9(S)-Indolizidin-3-One

D. 2(s)-Boc-(ACHP)-5(S)-Cyclopentyl-7-Oxa-9(S)-Indolizidin-3-One

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=10.50 min.

Partial 'H NMR (300 MHz, $CD_3OD$) $\delta$ = 1.42 (s, 9H), 2.63 (m, 1H), 3.06 (t, 1H), 3.39 (dd, 1H), 3.62 (dd, 1H), 3.69 (M, 1H), 3.88 (d, 1H), 3.8-4.0 (m, 3H).

E. 2(S)-Boc-(ACHP)-5(S)-Propyl-7-Oxa-9(S)-Indolizidin-3-One

The following are obtained using the procedure given in Example 2A, except Examples 2P, 2Q, 2Z, and 2FF, which require one additional equivalent of LDA and Example 2R, which requires two additional equivalents of LDA :

F. 2(S)-Boc-(ACHP)-7-Oxa-9(S)-Indolizidin-3-One

G. 2(S)-Boc-(ACHP)-5(S)-Vinyl-7-Oxa-9(S)-Indolizidin-3-One

H. 2(S)-Boc-(ACHP)-5(S)-Ethyl-7-Oxa-9(S)-Indolizidin-3-One

I. 2(S)-Boc-(ACHP)-5(S)-(2-Thieno)-7-Oxa-9(S)-Indolizidin-3-one

J. 2(S)-Boc-(ACHP)-5(S)-(2-Furyl)-7-Oxa-9(S)-Indolizidin-3-One

K. 2(S)-Boc-(ACHP)-5(S)-Phenyl-7-Oxa-9(S)-Indolizidin-3-One

L. 2(S)-Boc-(ACHP)-5(S)-Cyano-7-Oxa-9(S)-Indolizidin-3-One

N. 2(S)-Boc-(ACHP)-5(R)-Carbomethoxy-7-Oxa-9(S)-Indolizidin-3-One

P. 2(S)-Boc-(ACHP)-5(S)-(2-Hydroxyethyl)-7-Oxa-9(S)-Indolizidin-3-One

Q. 2(S)-Boc-(ACHP)-5(S)-[2-(2-Propylamino-carbamoyl)hydroxyethyl]-7-Oxa-9(S)-Indolizidin-3-One

R. 2(S)-Boc-(ACHP)-5(R)-Carboxamide-7-Oxa-9(S)-Indolizidin-3-One

S. 2(S)-Boc-(ACHP)-5(S)-(2-Diethylaminoethyl)-7-Oxa-9(S)-Indolizidin-3-One

T. 2-Boc-(ACHP)-9-Methyl-7-Oxa-Indolizidin-3-One

U. 2-Boc-(ACHP)-9-Ethyl-7-Oxa-Indolizidin-3-One

V. 6-Allyl-2(S)-Boc-(ACHP)-7-Oxa-9(S)-Indolizidin-3-One

W. 2(S)-Boc-(ACHP)-6-Phenyl-7-Oxa-9(S)-Indolizidin-3-One

X. 2(S)-Boc-(ACHP)-6-Vinyl-7-Oxa-9(S)-Indolizidin-3-One

Y. 2(S)-Boc-(ACHP)-6-Carboxy-7-Oxa-9(S)-Indolizidin-3-One

Z. 2(S)-Boc-(ACHP)-6-[(1-Methylpiperidin-3-yl)-Aminocarbonyl]-7-Oxa-9(S)-Indolizidin-3-One

AA. 2(S)-Boc-(ACHP)-6-(N,N-Diethylaminomethyl)-7-Oxa-9(S)-Indolizidin-3-One

BB. 5-Allyl-2-Boc-(ACHP)-9-Methyl-7-Oxa-Indolizidin-3-One

CC. 5(S)-Allyl-2(S)-Boc-(ACHP)-6-Methyl-7-Oxa-9(S)-Indolizidin-3-One

DD. 2(S)-Boc-(ACHP)-8-Methyl-7-Oxa-9(S)-Indolizidin-3-One

EE. 5(S)-Allyl-2(S)-Boc-(ACHP)-8-Methyl-7-Oxa-9(S)-Indolizidin-3-One

FF. 2(S)-Boc-(ACHP)-5(S)-Propyl-7-Hydroxy-9(S)-Indolizidin-3-One

GG. 2(S)-Boc-(ACHP)-5(S)-Propyl-6,7-Dehydro-9(S)-Indolizidin-3-One and 2(S)-Boc-(ACHP)-5(S)-Propyl-7,8-Dehydro-9(S)-Indolizidin-3-One

HH. 2(S)-Boc-(ACHP)-5(S)-Propyl-6-Oxa-8(S)-(Pyrrolizidine)-3-One

The following compounds are obtained by appropriate modification of the bicyclic lactam substituent(s) following coupling to 2(S)-Boc-(ACHP).

II. Preparation of 2(S)-Boc-(ACHP)-5(S)-Propyl-9(S)-Indolizidin-3,7-Dione

The title compound is prepared by oxidation of 2(S)-Boc-(ACHP)-5(S)-propyl-7-hydroxy-9(S)-indolizidin-3-one using pyridinium chlorochromate or Swern conditions.

KK. Preparation of 2(S)-Boc-(ACHP)-5(S)-Propyl-7-Methylene-9(S)-Indolizidin-3-One

The title compound is obtained by reaction of 2(S)-Boc-(ACHP)-5(S)-propyl-9(S)-indolizidin-3,7-dione with at least 3 equivalents of the Wittig reagent prepared by treatment of methyltriphenyl-phosphonium bromide with n-butyl lithium.

LL. Preparation of 2(S)-Boc-(ACHP)-7-Methyl-5(S)-Propyl-9(S)-Indolizidin-3-One

The title compound is prepared catalytic hydrogenation of 2(S)-Boc-(ACHP)-5(S)-propyl-7-methylene-9(S)-indolizidin-3-one using 10% Pd on carbon in EtOAc. The catalyst is removed by filtration, the filtrate solvent is removed under reduced pressure, and the residue is purified by chromatography on silica gel to give 2(S)-Boc-(ACHP)-7-methyl-5(S)-propyl-9(S)-indolizidin-3-one.

MM. Preparation of 2(S)-Boc-(ACHP)-7-(N,N-Diethyl-amino)-5(S)-Propyl-9(S)-Indolizidin-3-One

The title compound is obtained by reductive amination of 2(S)-Boc-(ACHP)-5(S)-propyl-9(S)-indolizidin-3,7-dione using diethylamine hydrochloride and $NaCNBH_3$.

NN. Preparation of 2(S)-Boc-(ACHP)-7-(2-Aminoethyl)-amino-5(S)-Propyl-9(S)-Indolizidin-3-One

The title compound is obtained by reductive amination of 2(S)-Boc-(ACHP)-5(S)-propyl-9(S)-indolizidin-3,7-dione using ethylenediamine dihydrochloride and $NaCNBH_3$.

OO. Preparation of 2(S)-Boc-(ACHP)-5(S)-Propyl-7-Methoxy-9(S)-Indolizidin-3-One

The title compound is prepared from 2(S)-Boc-(ACHP)-5(S)-propyl-7-hydroxy-9(S)-indolizidin-3-one by deprotonation with one equivalent of NaH followed by addition of methyl iodide.

PP. Preparation of 2(S)-Boc-(ACHP)-5(S)-Propyl-7-Epoxymethyl-9(S)-Indolizidin-3-One

The title compound is prepared by the reaction of 2(S)-Boc-(ACHP)-5(S)-propyl-9(S)-indolizidin-3,7-dione with dimethylsulfonium methylide (prepared in DMSO/THF according to the procedure of Corey and Chankovsky, J. Am. Chem. Soc., 1965, 87, 1353), or by epoxidation of 2(S)-Boc-(ACHP)-5(S)-propyl-7-methylene-9(S)-indolizidin-3-one with MCPBA.

QQ. Preparation of 2(S)-Boc-(ACHP)-5(S)-Propyl-7-(N-Ethylamino)methyl-7-Hydroxy-9(S)-Indolizidin-3-One and 2(S)-Boc-(ACHP)-5(S)-Propyl-7-(N-Ethylamino)-7-Hydroxymethyl-9(S)-Indolizidin-3-One

The title compounds are prepared by the reaction of 2(S)-Boc-(ACHP)-5(S)-propyl-7-epoxymethyl-9(S)-indolizidin-3-one with ethylamine hydrochloride.

RR. Preparation of 2(S)-Boc-(ACHP)-5(S)-Carboxymethyl-7-Oxa-9(S)-Indolizidin-3-One

Ozonolysis of 5(S)-allyl-2(S)-[2(S)-tert-butyloxycarbonylamino-3-cyclohexyl-1(S)-hydroxypropyl]-7-oxo-9(S)-indolizidin-3-one employing an oxidative workup with hydrogen peroxide gives the title compound.

SS. Preparation of 2(S)-Boc-(ACHP)-5(S)-Acetoxyethyl-7-Oxa-9(S)-Indolizidin-3-One

Acylation of 2(S)-Boc-(ACHP)-5(S)-2-hydroxyethyl-7-oxa-9(S)-indolizidin-3-one by treatment with acetic anhydride and pyridine gives the title compound.

TT. Preparation of 2(S)-Boc-(ACHP)-5(S)-(N-Quinuclidin-3-yl)carboxamidoethyl-7-Oxa-9(S)-Indolizidin-3-One

The title compound is prepared by the coupling of 2(S)-Boc-(ACHP)-5(S)-carboxymethyl-7-oxa-9(S)-indolizidin-3-one (obtained as described in Example 2 RR) with 3-aminoquinuclidine utilizing EDC in a DMF solution while maintaining pH 8 by the addition of DIPEA.

UU. Preparation of 2(S)-Boc-(ACHP)-5(S)-(N-Methyl-N-Ethyl)carboxamidomethyl-7-Oxa-9(S)-Indolizidin-3-One

The title compound is prepared by the coupling of 2(S)-Boc-(ACHP)-5(S)-carboxyethyl-7-oxa-9(S)-indolizidin-3-one with methylethylamine utilizing EDC in a DMF solution while maintaining pH 8 by the addition of DIPEA.

VV. Preparation of 2(S)-Boc-(ACHP)-5(S)-(2-Amino-ethyl-7-Oxa-9(S)-Indolizidin-3-One

The title compound is prepared by ozonolysis of 5(S)-allyl-2(S)-[2(S)-tert-butyloxycarbonylamino-3-cyclohexyl-1(S)-hydroxypropyl]-7-oxo-9(S)-indolizidin-3-one employing a reductive workup with dimethyl sulfide to give the aldehyde, followed by reductive amination with ammonia and $NaCNBH_3$.

WW. Preparation of 2(S)-Boc-(ACHP)-5(S)-2-[(Quinuclidin-3-yl)carbonylamino]ethyl-7-Oxa-9(S)-Indolizidin-3-One

The title compound is prepared by coupling 2(S)-Boc-(ACHP)-5(S)-(2-aminoethyl)-7-oxa-9(S)-indolizidin-3-one with quinuclidine-3-carboxylic acid utilizing EDC in a DMF solution while maintaining pH 8 by the addition of DIPEA.

The following was obtained using methodology as described :

XX. 2(S)-Boc-(ACHP)-6(S)-Methyl-9(S)-Indolizidin-3-one

Step 1 : Preparation of 1-Allyl-5(S)-(tert-Butyldimethylsilyl)oxymethyl-2-Pyrrolidinone

Protection of 1-allyl-5(S)-hydroxymethyl-2-pyrrolidinone as the TBDMS ether was effected by treatment with TBDMSCI and DMAP in $CH_2Cl_2$.

Step 2 : Preparation of 1-Allyl-3(S)-Boc-(ACHP)-5(S)-tert-Butyldimethylsilyloxymethyl-2-Pyrrolidinone

Aldol addition of 1-allyl-5(S)-tert-butyldimethylsilyloxymethyl-2-pyrrolidinone to Nα-Boc-L-cyclohexylalaninal was performed utilizing the procedure of Example 2A.

Step 3 : 2(S)-Boc-(ACHP)-6(S)-Methyl-9(S)-Indolizidin-3-One

Removal of the silyl ether from 1-allyl-3(S)-Boc-(ACHP)-5(S)-tert-butyldimethylsilyloxymethyl-2-pyrrolidinone by treatment with tetrabutylammonium fluoride in water/tetrahydrofuran, followed by oxidation with mercury trifluoroacetate and reduction with $NaBH_4$ in methanol gave 2(S)-Boc-(ACHP)-6-methyl-9(S)-indolizidin-3-one.

EXAMPLE 3

Amino acid coupling procedures and subsequent transformations

A. Preparation of Boc-Phe-His-(ACHP)-5(S)-Allyl-7-Oxa-9(S)-Indilizidin-3-One-2(S)-yl (Mixed Anydride Methodology

Step 1 : Boc-(DNP)His-(ACHP)-5(S)-Allyl-7-Oxa-9(S)-Indolizidin-3-One-2(S)-yl

To a solution of 5(S)-allyl-2(S)-[2(S)-tert-butyloxycarbonylamino-3-cyclohexyl-1(S)-hydroxypropyl]-7-oxa-9(S)-indolizidin-3-one (285 mg ; 0.654 mmol) in $CH_2Cl_2$ (3 mL) was added TFA (3 mL). The solution was stored under an atmosphere of nitrogen for 45 min at which time excess TFA and solvent were removed under reduced pressure. The oil so obtained was triturated in ether, giving a white solid which was collected by filtration, washed with ether, and dried under reduced pressure. The TFA salt (277 mg ; 94% yield) was dissolved under an atmosphere of nitrogen in dry, degassed DMF (3 mL) and stored while the activation of Boc(DNP)His-OH was accomplished by in situ formation of a mixed anhydride as described below. To a suspension of Boc(DNP)His-OH (440 mg ; 1.05 mmol) in dry EtOAc (6 mL) under an atmosphere of nitrogen was added NMM (150 µL ; 1.36 mmol), which caused dissolution of any remaining solid. The solution was cooled to -23°C and isobutylchloroformate (136 µL ; 1.05 mmol) was added, and the resulting suspension was stirred at -23°C for 20 minutes. At this time the DMF solution of the TFA salt was neutralized by the addition of NMM (93 µL ; 850 mmol) and the resulting solution was added via cannula to the cold solution of the mixed anhydride. After being stirred for 1 hour at -23°C, the reaction mixture was warmed to 0°C and was stirred for 2 hours. The cooling bath was removed and stirring was continued for another 3 hours, at which time the reaction was quenched by the addition of water (10 mL). The mixture was diluted with EtOAc (50 mL) and was washed successively with 5% aqueous HCl (25 mL), water (10 mL), and saturated aqueous $NaHCO_3$ (25 mL). The organic layer was dried ($MgSO_4$), filtered, and concentrated under reduced pressure to give an orange solid which was flash chromatographed over silica gel using a gradient elution of 3% to 5% $MeOH/CH_2Cl_2$. The coupling product was obtained as a yellow solid (367 mg ; 76% yield).

Step 2 : Boc-Phe-(DNP)His-(ACHP)-5(S)-Allyl-7-Oxa-9(S)-Indolizidin-3-One-2(S)-yl

To a solution of the coupling product obtained from step 1 above (367 mg ; 497 mmol) in $CH_2Cl_2$ (3 mL) was added TFA (3 mL) and the mixture was stored under an atmosphere of nitrogen for 45 min, at which time the solvent and excess TFA were removed under reduced pressure. The yellow oil was triturated in ether and the solid which resulted was collected by filtration, washed with ether, and dried under reduced pressure. The orange-yellow TFA salt (344 mg ; 92% yield) was dissolved under an atmosphere of nitrogen in dry EtOAc (3 mL) and stored while the activation of Boc-Phe-OH was accomplished by in situ formation of a mixed anhydride as described below. To a solution of Boc-Phe-OH (211 mg ; 0.795 mmol) in dry EtOAc (5 mL) under an atmosphere of nitrogen was added NMM (114 µL ; 1.03 mmol). The resulting solution was cooled to -23°C, when isobutylchloroformate (103 µL ; 0.795 mmol) was added, and the solution was stirred for 25 min. At this time, the solution of the TFA salt was neutralized by the addition of NMM (42 µL ; 382 mmol) and the resulting solution was added via cannula to the cold solution of the mixed anhydride. The resulting mixture was stirred at -23°C for 1 hour, at 0°C for 2 hours, and then at ambient temperature for 3 hours. The reaction was quenched by the addition of water (10 mL) and was diluted with EtOAc (50 mL). The organic phase was washed successively with 5% aqueous HCl (25 mL), water (20 mL), and saturated aqueous $NaHCO_3$ (25 mL), then dried ($MgSO_4$), and filtered. Removal of the solvent under reduced pressure gave an orange solid which was flash chromatographed over silica gel using a gradient elution of 4% to 7% $MeOH/CH_2Cl_2$. The coupling product was obtained as a yellow solid (390 mg ; 88% yield).

Step 3 : Boc-Phe-His-(ACHP)-5(S)-Allyl-7-Oxa-9(S)-Indolizidin-3-One-2(S)-yl

To a solution of the coupling product obtained from step 2 above (390 mg ; 0.437 mmol) in $CH_2Cl_2$ (2 mL) was added thiophenol (2 mL) and NMM (20 µL ; 0.18 mmol). The mixture was stirred at room temperature for 4 hours, at which time the solvent and excess thiophenol were removed under reduced pressure (0.2 torr) at 40°C. The residue was flash chromatographed over silica gel using a gradient elution of 5% to 10% $MeOH/CH_2Cl_2$. The product was lyophilized from dioxane and triturated in 1 :1 ether-hexane to give the title compound as an off-white powder (275 mg ; 88%).

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=7.68 min.

Partial 'H NMR (300 MHz, $CD_3OD$) δ = 1.35 (S, 9H), 1.47 (M, 1H), 2.25 (ddd, 1H), 2.4-2.5 (M, 2H), 2.58 (M, 1H), 2.88 (dd, 1H), 3.0-3.2 (M, 3H), 3.39 (dd, 1H), 3.78 (d, 1H), 3.84 (dd, 1H), 3.85-4.0 (M, 3H), 4.03 (M, 1H), 4.28 (dd, 1H) 4,53 (t, 1H), 5.04 (dm, 1H) 5.12 (dd, 1H), 5.80 (ddt, 1H), 6.91 (s, 1H), 7.2-7.3 (M, 5H), 7.58 (d, 1H).

Anal. Calcd. for $C_{39}H_{56}N_6O_7•H_2O$ : C, 63.39 ; H, 7.91 ; N, 11.37 ; Found : C, 63.70 ; H, 7.82 ; N, 11.42.

Inhibition of human plasma renin was assayed using the in vitro method described by Boger, et. al., J. Med. Chem. (1985) 28, 1779. $IC_{50}$-1.3 nM.

B. Preparation of Boc-Phe-His-(ACHP)-5(S)-(2-Methallyl)-7-Oxa-9(S)-Indolizidin-3-One-2(S)-yl

Amino acid coupling was accomplished as described in Example 3A.

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 30 min ; 2.0 ml/min) RT=18.06 min.

Partial 'H NMR (360 MHz, $CD_3OD$) 1.35 (s, 9H), 1.47 (m, 1H), 1.77 (s, 3H), 2.25 (ddd, 1H), 2.38-2.48 (m, 2H), 2.57 (m, 1H), 2.78 (dd, 1H), 2.97-3.15 (m, 3H), 3.08 (t, 1H), 3.40 (dd, 1H), 3.78 (d, 1H), 3.85 (dd, 1H), 3.90 (m, 1H), 3.95 (dd, 1H), 4.04 (m, 1H), 4.12 (dt, 1h), 4.28 (dd, 1H), 4.55 (t, 1H), 4.77 (brs, 1H), 4.80 (brs, 1H), 6.92 (s, 1H), 7.2-7.35 (m, 5H), 7.60 (d, 1H).

Anal. Calcd. For $C_{40}H_{58}N_6O_7 \bullet H_2O$) : C, 63.81 ; H, 8.03 ; N, 11.16 ; Found : C, 63.94 ; H, 7.91 ; N, 11.09.

$IC_{50}$ = 0.97 nM.

C. Preparation of Boc-Phe-His-(ACHP)-5(S)-(2-Methylpropyl)-7-Oxa-9(S)-Indolizidin-3-One-2(S)-yl

Amino acid coupling was accomplished as described in Example 3A.

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 30 min ; 2.0 ml/min) RT=18.50 min.

Partial 'H NMR (360 MHz, $CD_3OD$) δ = 0.91 (d, 3H), 0.95 (d, 3H), 1.34 (s, 9H), 2.25 (ddd, 1H), 2.61 (m, 1H), 2.78 (dd, 1H), 2.98-3.15 (m, 3H), 3.05 (t, 1H), 3.44 (dd, 1H), 3.69 (d, 1H), 3.82 (m, 1H), 3.87 (dd, 1H), 3.92 (dd, 1H), 4.03 (m, 2H)), 4.27 (dd, 1H), 4.53 (t, 1H), 6.92 (s, 1H), 7.15-7.20 (m, 5H), 7.58 (s, 1H).

Anal. Calcd. For $C_{40}H_{60}N_6O_7 \bullet \frac{1}{2}dioxane \bullet \frac{1}{2}H_2O$ : C, 63.86 ; H, 8.29 ; N, 10.64 ; Found : C, 64.01 ; H, 7.87 ; N, 10.88.

$IC_{50}$ = 2.0 nM.

D. Preparation of Boc-Phe-His-(ACHP)-5(S)-Cyclopentyl-7-Oxa-9(S)-Indolizidin-3-One-2(S)-yl

Amino acid coupling was accomplished as described in Example 3A.

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 30 min ; 2.0 ml/min) RT=18.50 min.

Partial 'H NMR (360 MHz, $CD_3OD$) δ = 2.27 (ddd, 1H), 2.51 (M, 1H), 2.61 (m, 1H), 2.78 (dd, 1H), 2.95-3.15 (m, 3H), 3.06 (t, 1H), 3.40 (dd, 1H), 3.63 (dd, 1H), 3.86 (d, 1H), 3.8-3.9 (m, 2H), 3.95 (dd, 1H), 4.04 (m, 1H), 4.28 (dd, 1H), 4.54 (t, 1H), 6.94 (s, 1H), 7.2-7.3 (m, 5H), 7.59 (s, 1H).

Anal. Calcd. For $C_{41}H_{60}N_6O_7 \bullet H_2O$ : C, 64.21 ; H, 8.15 ; N, 10.96 ; Found : C, 64.61 ; H, 8.13 ; N, 10.77.

$IC_{50}$ = 1.0 nM.

E. Preparation of Boc-Phe-His-(ACHP)-5(S)-Propyl-7-Oxa-9(S)-Indolizidin-3-One-2(S)-yl

Amino acid coupling was accomplished as described in Example 3A.

HPLC (Vydac $C_{18}$ reverse phase column, 12cm ; gradient from 95% $H_2O$ (0.1% TFA) : 5% $CH_3CN$ (0.1% TFA) to 100% $CH_3CN$ (0.1% TFA) over 15 min ; 2.0 ml/min) RT=7.75 min.

Partial 'H NMR (300 MHz, $CD_3OD$) δ = 0.93 (t, 1H), 1.34 (s, 9H), 2.26 (M, 1H), 2.60 (M, 1H), 2.78 (dd, 1H), 3.05 (t, 1H), 3.00-3.15 (M, 3H), 3.42 (dd, 1H), 3.73 (d, 1H), 3.80-3.95 (M, 2H), 3.87 (dd, 1H), 3.92 (dd, 1H), 4.02 (M, 1H), 4.28 (dd, 1H), 4.53 (t, 1H), 6.91 (s, 1H), 7.15-7.30 (m, 5H), 7.55 (d, 1H).

Anal. Calcd. for $C_{39}H_{58}N_6O_7 \bullet H_2O$ : C, 63.32 ; H, 8.16 ; N, 11.34 ; Found : C, 63.14 ; H, 8.14 ; N, 11.34.

$IC_{50}$ = 1.0 nM.

XX. Preparation of Boc-Phe-His-(ACHP)-6(S)-Methyl-9(S)-Indolizidin-3-One-2(S)-yl

Amino acid coupling was accomplished as described in Example 3A. $IC_{50}$ = 6.8 nM.

The following peptides are obtained using the procedure given in Example 3A :

F. Boc-Phe-His-(ACHP)-7-Oxa-9(S)-Indolizidin-3-One-2(S)-yl

G. Boc-Phe-His-(ACHP)-5(S)-Vinyl-7-Oxa-9(S)-Indolizidin-3-One-2(S)-yl

H. Boc-Phe-His-(ACHP)-5(S)-Ethyl-7-Oxa-9(S)-Indolizidin-3-One-2(S)-yl

I. Boc-Phe-His-(ACHP)-5(R)-(2-Thieno)-7-Oxa-9(S)-Indolizidin-3-One-2(S)-yl

J. Boc-Phe-His-(ACHP)-5(R)-(2-Furyl)-7-Oxa-9(S)-Indolizidin-3-One-2(S)-yl

K. Boc-Phe-His-(ACHP)-5(S)-Phenyl-7-Oxa-9(S)-Indolizidin-3-One-2(S)-yl

L. Boc-Phe-His-(ACHP)-5(S)-Cyano-7-Oxa-9(S)-Indolizidin-3-One-2(S)-yl

N. Boc-Phe-His-(ACHP)-5(S)-Carboxyethyl-7-Oxa-9(S)-Indolizidin-3-One-2(S)-yl

P. Boc-Phe-His-(ACHP)-5(S)-(2-Hydroxyethyl)-7-Oxa-9(S)-Indolizidin-3-One-2(S)-yl

Q. Boc-Phe-His-(ACHP)-5(S)-[2-(2-Propylamino-carbamoyl)hydroxyethyl]-7-Oxa-9(S)-Indolizidin-3-One-2(S)-yl

R. Boc-Phe-His-(ACHP)-5(R)-Carboxamide-7-Oxa-9(S)-Indolizidin-3-One-2(S)-yl

S. Boc-Phe-His-(ACHP)-5(S)-(2-Diethylamino-ethyl)-7-Oxa-9(S)-Indolizidin-3-One-2(S)-yl

T. Boc-Phe-His-(ACHP)-9-Methyl-7-Oxa-Indolizidin-3-One-2(S)-yl

U. Boc-Phe-His-(ACHP)-9-Ethyl-7-Oxa-Indolizidin-3-One-2(S)-yl

V. Boc-Phe-His-(ACHP)-6-Allyl-7-Oxa-9(S)-Indolizidin-3-One-2(S)-yl

W. Boc-Phe-His-(ACHP)-6-Phenyl-7-Oxa-9(S)-Indolizidin-3-One-2(S)-yl

X. Boc-Phe-His-(ACHP)-6-Vinyl-7-Oxa-9(S)-Indolizidin-3-One-2(S)-yl

Y. Boc-Phe-His-(ACHP)-6-Carboxy-7-Oxa-9(S)-Indolizidin-3-One-2(S)-yl

Z. Boc-Phe-His-(ACHP)-6-[(1-Methylpiperidin-3-yl)aminocarbonyl]-7-Oxa-9(S)-Indolizidin-3-One-2(S)-yl

AA. Boc-Phe-His-(ACHP)-6-(N,N-Diethylamino-methyl)-7-Oxa-9(S)-Indolizidin-3-One-2(S)-yl

BB. Boc-Phe-His-(ACHP)-6-Allyl-9-Methyl-7-Oxa-Indolizidin-3-One-2(S)-yl

CC. Boc-Phe-His-(ACHP)-5(S)-Allyl-6-Methyl-7-Oxa-Indolizidin-3-One-2(S)-yl

DD. Boc-Phe-His-(ACHP)-8-Methyl-7-Oxa-9(S)-Indolizidin-3-One-2(S)-yl

EE. Boc-Phe-His-(ACHP)-5(S)-Allyl-8-Methyl-7-Oxa-9(S)-Indolizidin-3-One-2(S)-yl

FF. Boc-Phe-His-(ACHP)-5(S)-Propyl-7-Hydroxy-9(S)-Indolizidin-3-One-2(S)-yl

GG. Boc-Phe-His-(ACHP)-5(S)-Propyl-6,7-Dehydro-9(S)-Indolizidin-3-One-2(S)-yl and Boc-Phe-His-(ACHP)-5(S)-Propyl-7,8-Dehydro-9(S)-Indolizidin-3-One-2(S)-yl

HH. Boc-Phe-His-(ACHP)-5(S)-Propyl-6-Oxa-8(S)-(Pyrrolizidine)-3-One-2(S)-yl

II. Boc-Phe-His-(ACHP)-5(S)-Propyl-9(S)-Indolizidin-3,7-Dione-2(S)-yl

KK. Boc-Phe-His-(ACHP)-5(S)-Propyl-7-Methylene-9(S)-Indolizidin-3-One-2(S)-yl

LL. Boc-Phe-His-(ACHP)-7-Methyl-5(S)-Propyl-9(S)-Indolizidin-3-One-2(S)-yl

MM. Boc-Phe-His-(ACHP)-7-(N,N-Diethylamino-5(S)-Propyl-9(S)-Indolizidin-3-One-2(S)-yl

NN. Boc-Phe-His-(ACHP)-7-(2-Aminoethyl)amino-5(S)-Propyl-9(S)-Indolizidin-3-One-2(S)-yl

OO. Boc-Phe-His-(ACHP)-5(S)-Propyl-7-Methoxy-9(S)-Indolizidin-3-One-2(S)-yl

QQ. Boc-Phe-His-(ACHP)-5(S)-Propyl-7-(N-Ethylamino)methyl-7-Hydroxy-9(S)-Indolizidin-3-One-2(S)-yl and Boc-Phe-His-(ACHP)-5(S)-Propyl-7-(N-Ethylamino)-7-Hydroxymethyl-9(S)-Indolizidin-3-One-2(S)-yl

RR. Boc-Phe-His-(ACHP)-5(S)-Carboxymethyl-7-Oxa-9(S)-Indolizidin-3-One-2(S)-yl

SS. Boc-Phe-His-(ACHP)-5(S)-2-Acetoxyethyl-7-Oxa-9(S)-Indolizidin-3-One-2(S)-yl

TT. Boc-Phe-His-(ACHP)-5(S)-(N-Quinuclidin-3-yl)-carboxamidoethyl-7-Oxa-9(S)-Indolizidin-3-One-2(S)-yl

UU. Boc-Phe-His-(ACHP)-5(S)-(N-Methyl-N-Ethyl)-carboxamidomethyl-7-Oxa-9(S)-Indolizidin-3-One-2(S)-yl

VV. Boc-Phe-His-(ACHP)-5(S)-(2-Aminoethyl-7-Oxa-9(S)-Indolizidin-3-One-2(S)-yl

WW. Boc-Phe-His-(ACHP)-5(S)-2-[(Quinuclidin-3-yl)carbonylamino]ethyl-7-Oxa-9(S)-Indolizidin-3-One-2(S)-yl

JJ. Boc-Phe-His-(ACHP)-7-Hydroxyimino-5(S)-Propyl-9(S)-Indolizidin-3-One-2(S)-yl

The title compound is prepared by reaction of Boc-Phe-His-(ACHP)-5(S)-propyl-9(S)-indolizidin-3,7-dione-2(S)-yl with hydroxylamine hydrochloride in the presence of pyridine.

Other examples of lactam modification after the peptide segment has been attached are given by the following examples.

Ipoc-Phe-His- peptides are made using sequential coupling, using Ipoc-Phe instead of Boc-Phe, in the method according to Example 3A.

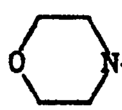 -CO-Phe-His- peptides are made by

sequential coupling using mixed anhydride coupling according to Example 3A for the histidine group, and using mixed anhydride or DCC/HOBT coupling for the

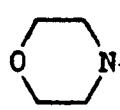 -CO-Phe group.

$(CH_3)_2CH-SO_2$-Phe-His-peptides are made by sequential coupling using mixed anhydride coupling according to Example 3A for the histidine group, and using mixed anhydride or DCC/HOBT coupling for the $(CH_3)_2CH-SO_2$-Phe group.

$(CH_3)_2CH-SO_2-CH_2-CH(CH_2Ph)$-CO-His- peptides are made by sequential coupling using mixed anhydride coupling according to Example 3A for the histidine group and DCC/HOBT or DCC/HOSU coupling for the $(CH_3)_2CH-SO_2-CH_2-CH(CH_2Ph)$-CO- group.

2-Indolyl-CO-His- peptides are made by sequential coupling using mixed anhydride coupling according to Example 3A for the histidine group, and DCC or EDC coupling of indole-2-carboxylic acid.

These methods are applied to any of the 47 Boc-(ACHP)-bicyclic lactams described in Example 2.

The following examples are illustrative of the methods for producing quaternary ammonium salts. Purification of these compounds is accomplished using preparative reverse phase HPLC, using acetonitrile/water containing 0.1% TFA as an eluent. The final products are obtained as acetate salts by passage through an ion exchange column (Bio-Rad AG3-X4A resin, acetate form).

YY. Boc-Phe-His-(ACHP)-5(S)-[2-(Quinuclidin-1-yl)-ethyl]-7-Oxa-9(S)-Indolizidin-3-One-2(S)-yl Acetate

Boc-Phe-His-(ACHP)-5(S)-(2-hydroxyethyl)-7-oxa-9(S)-indolizidin-3-one is brominated by treatment with $Ph_3P/CBr_4$ in THF solution to give Boc-Phe-His-(ACHP)-5(S)-(2-bromoethyl)-7-oxa-9(S)-indolizidin-3-one. Reaction of the latter with quinuclidine in DMF solution gives the quaternary ammonium bromide salt, which

with purification and ion exchange, as described above, gives the title compound.

ZZ. Boc-Phe-His-(ACHP)-5(S)-(2-(Triethylamino)ethyl]-7-Oxa-9(S)-Indolizidin-3-One-2(S)-yl

Boc-Phe-His-(ACHP)-5(S)-(2-diethylaminoethyl)-7-oxa-9(S)-indolizidin-3-one-2(S)-yl is protected on the histidine side chain with $(Boc)_2O$ in DMF solution in the presence of DIPEA. Ethyl iodide (1.1 equivalent) and $Na_2CO_3$ are then added and the solution is warmed to 50°C.

When the reaction is complete by TLC, $H_2O$ and $Et_3N$ (5 equivalents each) are added to remove the Boc group, with purification and ion exchange, as described above, giving the title compound.

AAA. Ipoc-Phe-His-(ACHP)-5(S)-1-[N-Benzyl-(2-Diethyl-Aminoethyl)]-7-Oxa-9(S)-Indolizidin-3-One-2(S)-yl

Ipoc-Phe-His-(ACHP)-5(S)-(2-diethylaminoethyl)-7-oxa-9(S)-indolizidin-3-One-2(S)-yl is protected on the histidine side chain with $(Boc)_2O$ in DMF solution in the presence of DIPEA. Benzyl bromide (1 equivalent) and $Na_2CO_3$ are then added and the solution is warmed to 50°C.

When the reaction is complete by TLC, $H_2O$ and $Et_3N$ (5 equivalents each) are added to remove the Boc group, with purification and ion exchange, as described above, giving the title compound.

BBB. Boc-Phe-His-(ACHP)-6(S)-[(1,1-Dimethylpiperidinium-3-yl)aminocarbonyl]-7-oxa-9(S)-Indolizidin-3-One-2(S)-yl

Boc-Phe-His-(ACHP)-6-[(1-methylpiperidin-3-yl)aminocarbonyl]-7-oxa-9(S)-indolizidin-3-one-2(S)-yl is protected on the histidine side chain with $(Boc)_2O$ in DMF solution in the presence of DIPEA. Methyl iodide (1 equivalent) and $Na_2CO_3$ are then added and the solution is warmed to 50°C.

When the reaction is complete by TLC, $H_2O$ and $Et_3N$ (5 equivalents each) are added to remove the Boc group, with purification and ion exchange, as described above, giving the title compound.

CCC. Boc-Phe-His-(ACHP)-7-[(N-Methyl-N,N-Diethyl)-Amino]-5(S)-Methyl-9(S)-Indolizidin-3-One-2(S)-yl

Boc-Phe-His-(ACHP)-7-(N,N-diethylamino)-5(S)-methyl-9(S)-indolizidin-3-one is protected on the histidine side chain with $(Boc)_2O$ in DMF solution in the presence of DIPEA. Ethyl iodide (1 equivalent) and $Na_2CO_3$ are then added and the solution is warmed to 50°C.

When the reaction is complete by TLC, $H_2O$ and $Et_3N$ (5 equivalents each) are added to remove the Boc group, with purification and ion exchange, as described above, giving the title compound.

While the foregoing specification teaches the principles of the present invention, with examples provided for the purpose of illustration, it will be understood that the practice of the invention encompasses all of the casual variations, adaptations, modifications, deletions, or additions of procedures and protocols described herein, as come within the scope of the following claims and its equivalents.

**Claims**

1. A peptide of the formula :

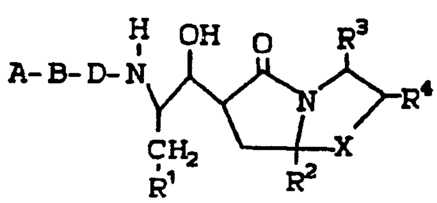

wherein :

A is hydrogen ;
$C_1$-$C_6$-alkyl ;
aryl, where aryl is unsubstituted or mono-, di- or trisubstituted phenyl, wherein the substituent(s) is/are independently selected from the group consisting of $C_1$-$C_4$-alkyl, phenyl-$C_1$-

$C_4$-alkyl, amino, mono- or di-$C_1$-$C_4$-alkylamino, amino-$C_1$-$C_4$-alkyl, hydroxy-$C_1$-$C_4$-alkyl, mono- or di-$C_1$-$C_4$- alkylamino-$C_1$-$C_4$-alkyl, guanidyl, guanidyl-$C_1$-$C_4$-alkyl, hydroxyl,

$C_1$-$C_4$-alkoxy, trifluoromethyl, halo, CHO, $-CO_2H$, $-CONH_2$, -CO-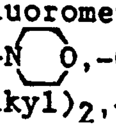, $-CONH-C_1-C_4$-alkyl, $-CON(C_1-C_4\text{-alkyl})_2$, $-CO-C_1-C_4$-alkyl,

$-(CH_2)_m-{}^+N(R^5)_2R^6$ $A^-$, where $R^5$ is $C_1$-$C_4$-alkyl ; $-(CH_2)_p-$, wherein p is 4-to-6 ; or $-(CH_2)_2-O-(CH_2)_2-$ ; $R^6$ is $C_1$-$C_4$-alkyl, hydroxy-$C_1$-$C_4$-alkyl, carboxy-$C_1$-$C_4$-alkyl, carbo-$C_1$-$C_4$- alkoxy-$C_1$-$C_4$-alkyl, or-$CH_2$-phenyl ;

$A^-$ is a counterion selected from the group consisting of single negatively-charged ions, such as chloride, bromide, perchlorate, benzoate, benzene sulfonate, tartrate, maleate, hemitartate, and acetate ;

and m is 0-to-3; $-CO_2-C_1-C_4$-alkyl, $-CO_2-C_1-C_4$-alkoxy-$C_2$-$C_4$-alkyl, $-(CH_2)_m-{}^+$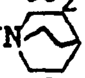 $A^-$, where $A^-$ and m are as defined above, and

$-NR^7R^8$, where $R^7$ and $R^8$ are independently hydrogen or unsubstituted or monosubstituted $C_1$-$C_4$-alkyl, wherein the substituent is amino, mono- or di-$C_2$-$C_4$-alkylamino or $-{}^+N(R^5)_2R^6$ $A^-$, where $R^5$, $R^6$ and $A^-$ are as defined above ;

Het, where Het is an unsubstituted or mono-or disubstituted 5-to-7-membered monocyclic or 7-to-10-membered bicyclic heterocyclic ring, wherein the one or two heteroatoms are independently selected from the group consisting of N, O, S, NO, SO, $SO_2$ and quaternized N, and the substituent(s), when attached to carbon atom(s) in the heterocyclic ring, is/are independently selected from the group consisting of hydroxyl, thiol, $C_1$-$C_6$-alkyl, $CF_3$, aryl- $C_1$-$C_4$-alkoxy, halo, aryl or $C_1$-$C_4$-alkyl, where aryl is as defined above, amino, mono- or di-$C_1$-$C_4$-alkylamino, amino-$C_1$-$C_4$-alkyl, hydroxy-$C_1$-$C_4$-alkyl, mono- or di-$C_1$-$C_4$-alkyl- amino-$C_1$-$C_4$-alkyl, guanidyl, guanidyl-$C_1$-$C_4$- alkyl, CHO, $CO_2H$, $CO_2$-$C_1$-$C_4$-alkyl, $CONH_2$,

$CONH-C_1-C_4$-alkyl, $CON(C_1-C_4\text{-alkyl})_2$, -CO-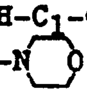, $NR^7R^8$, $-(CH_2)_m-{}^+$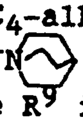 $A^-$ and $-(CH_2)_m-{}^+N(R^5)_2R^9$ $A^-$, where $R^9$ is

$C_1$-$C_4$-alkyl, hydroxy-$C_1$-$C_4$-alkyl, carboxy-$C_1$-$C_4$-alkyl, carbo-$C_1$-$C_4$-alkoxy- $C_1$-$C_4$-alkyl, or $-CH_2$-phenyl, wherein $R^7$, $R^8$, $A^-$, m and $R^5$ are as defined above, or, when attached to $sp^3$ hybridized heteroatom nitrogen(s) in the heterocycle ring, is/are independently selected from the group consisting of hydrogen ; unsubstituted or mono-substituted $C_1$-$C_7$-alkyl, where the substituent is independently selected from the group consisting of hydroxyl, amino, mono- or di-$C_1$-$C_4$-alkyl-amino, $-CO_2H$,

$-CONH_2$, $-CO-NH-C_1-C_4$-alkyl, $-CON(C_1-C_4\text{-alkyl})_2$, -CO-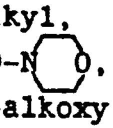, -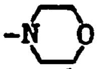, $CO_2-C_1-C_4$-alkyl, $C_1$-$C_7$-alkoxy, and aryl, as

defined above ; $-CO-C_1-C_4$-alkyl ; $-CO_2-C_1-C_7$-alkyl ; $-CO-NH-C_1-C_7$-alkyl ; $-SO_2-C_1-C_7$-alkyl ; -CHO ; and -CO-aryl, -CO-NH-aryl or $-SO_2$-aryl, where aryl is as defined above ; or, when attached to a quaternized $sp^3$ hybridized nitrogen in the heterocyclic ring, are independently selected from the group consisting of $C_1$-$C_7$-alkyl and mono-substituted $C_1$-$C_4$-alkyl, where the substituent is independently selected from the group consisting of hydroxyl, $-CO_2H$, $-CO_2-C_1-C_4$-

alkyl, $-SO_3H$, $-SO_2NH_2$, $C_1$-$C_4$-alkoxy, di-$C_1$-$C_4$-alkylamino, 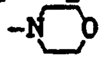, and aryl, as defined above; or, are alternatively

$-(CH_2)_q$- or $-(CH_2)_2-O-(CH_2)_2$- and form a quaternary spirocyclic ring with the $sp^3$ hybridized N-atom, wherein q is 3-to-6 ; or, when attached to a quaternized $sp^2$ hybridized nitrogen in the heterocyclic ring, are independently selected from the group consisting of $C_1$-$C_7$-alkyl and mono-substituted $C_1$-$C_4$-alkyl, where the substituent is independently selected from the group consisting of hydroxyl, $C_1$-$C_4$-alkoxy, $-CO_2H$ and $-CO_2$-$C_1$-$C_4$-alkyl ;

$$R^{10}-\overset{\overset{\displaystyle O}{||}}{C}-,$$

where $R^{10}$ is $C_1$-$C_7$-alkyl ;
hydrogen ;
Het, as defined above ;
aryl, as defined above ;
mono-substituted $C_1$-$C_6$-alkyl, wherein the substituent is selected from the group consisting of aryl, as defined above ; Het, as defined above ; hydroxyl ; $C_1$-$C_4$-alkoxy ; $C_3$-$C_7$-cycloalkyl ; amino ; mono- or di-$C_1$-$C_4$-alkyl-amino ; Het-$C_1$-$C_4$-alkyl, wherein Het is as defined above ; aryl or aryl-$C_1$-$C_4$- alkyl, wherein aryl is as defined above ; $-CO_2H$ ;
$-CO_2R^{11}$, where $R^{11}$ is $C_1$-$C_5$-alkyl, aryl, as defined above, Het, as defined above, mono-substituted $C_2$-$C_5$-alkyl, wherein the substituent is hydroxyl, $C_1$-$C_4$-alkoxy, $C_1$-$C_5$-alkyl-$CO_2$-, $C_1$-$C_5$-alkyl-CONH-, H-CONH-, amino, mono- or dialkylamino or halo ; $-CONH_2$ ; $-CONH-R^{11}$ or $-S(O)_n-R^{11}$, wherein n is 0-to-2 and $R^{11}$ is as defined above ; $-NH-CO-R^{11}$, where $R^{11}$ is as defined above ; -NH-aryl, $-NH-CH_2$- aryl or -CO-aryl, where aryl is as defined above ; and -NH-Het, $-NH-CH_2$-Het or -CO-Het, where Het is as defined above ;

$$R^{11}-O-\overset{\overset{\displaystyle O}{||}}{C} \text{ or } R^{11}-NH-\overset{\overset{\displaystyle O}{||}}{C},$$

where $R^{11}$ is as defined above ; or

$$R^{12}-\underset{\underset{\displaystyle O}{||}}{\overset{\overset{\displaystyle O}{||}}{S}}-,$$

where $R^{12}$ is independently selected from the definitions of $R^{11}$, $C_6$-or-$C_7$- alkyl, or Het, as defined above;
B and D are independently

$$-\overset{\overset{\displaystyle R^{13}}{|}}{N}-\underset{\underset{\displaystyle R^{10}}{|}}{CH}-\overset{\overset{\displaystyle O}{||}}{C},$$

where $R^{13}$ is hydrogen, $C_1$-$C_5$-alkyl, or $-CH_2$-aryl, wherein aryl is as defined above ; and $R^{10}$ is as defined above ;

or 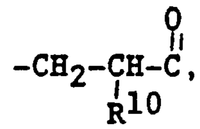

where $R^{10}$ is as defined above ; or either B or D, but not both simultaneously, is absent ;

$R^1$ is hydrogen ;
$C_3$-$C_6$-alkyl ;
aryl, as defined above ;
unsubstituted, mono-, di- or trisubstituted $C_3$-$C_7$-cycloalkyl, where the substituent(s) is/are selected from the group consisting of $C_1$-$C_4$-alkyl, trifluoromethyl, hydroxyl, $C_1$-$C_4$-alkoxy and halo ; or unsubstituted or 4-monosubstituted 1,3-dithiolan-2-yl or unsubstituted or 4-monosubstituted 1,3-dithian-2-yl, where the substituent is $-(CH_2)_m$-aryl, where m and aryl are as defined above ;

$R^2$ is hydrogen, $C_1$-$C_7$-alkyl, $C_2$-$C_7$-alkenyl, phenyl or $C_1$-$C_3$-alkyl-phenyl ;

X is $-CH_2-$ ; -O- ; $-CH_2CH_2-$ ; -CH=CH- ; $-CH_2NH-$ :

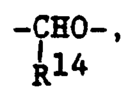

where $R^{14}$ is hydrogen or $C_1$-$C_7$-alkyl ;

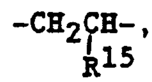

where $R^{15}$ is $C_1$-$C_6$ alkyl, hydroxyl, $C_1$-$C_4$ alkoxyl, $C_1$-$C_6$ acyloxy, amino, mono-$C_1$-$C_6$-alkylamino, di-$C_1$-$C_6$-alkylamino, amino-$C_1$-$C_5$-alkyl, mono-$C_1$-$C_6$-alkylamino- $C_1$-$C_6$-alkyl, $C_1$-$C_4$ alkoxylthio-$C_1$-$C_4$-alkyl, fluoro, carboxy, carboxy-$C_1$-$C_6$-alkylamido, aryl or aryl $C_1$-$C_4$ alkoxyl, where aryl is as defined above ;

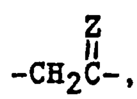

where Z is oxo, $-OCH_2-$, $C_1$-$C_6$ alkyl imino, methylene, $C_1$-$C_6$ alkylmethylene, ;

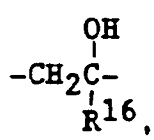

where $R^{16}$ is aminomethyl, mono or di-$C_1$-$C_5$-alkyl-aminomethyl, 4-morpholino, 1-pyrrolidinylmethyl, 1-piperidinylmethyl ;

$R^3$ is hydrogen ; aryl or -CO-aryl, where aryl is as defined above ; -CO-Het, where Het is as defined above ; $-CO_2H$ ; -CO-NH-$R^{11}$ or -CO-N($R^{13}$)-$R^{11}$, where $R^{13}$ and $R^{11}$ are as defined above ; or unsubstituted or mono- substituted $C_1$-$C_8$-alkyl or -CO-$C_1$-$C_8$-alkyl, $C_2$-$C_8$-alkenyl, $C_3$-$C_7$-cycloalkyl or $C_5$-$C_7$-cycloalkenyl, where the substituent on the $C_1$-$C_8$-alkyl, $C_2$-$C_8$-alkenyl, $C_3$-$C_7$-cycloalkyl or $C_5$-$C_7$-cycloalkenyl is selected from the group consisting of $C_1$-$C_7$-alkyl, $C_3$-$C_7$-cyclo-alkyl, hydroxyl, halo, -CHO, $-CO_2H$, $-CO_2R^{11}$ or -CONH-$R^{11}$ or -NHCO-$R^{11}$, wherein $R^{11}$ is as defined above, -O-CO-$R^{12}$, wherein $R^{12}$ is as defined above, amino, mono- or di-$C_1$-$C_4$-alkylamino, mono- amino-$C_1$-$C_4$-alkylamino, $-NHR_{11}$, -N($R^{13}$)-CO-$R^{12}$ or -CON($R^{13}$)-$R^{11}$, wherein $R^{13}$, $R^{12}$ and $R^{11}$ are as defined above,

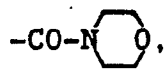

and aryl, as defined above ;
$R^4$ is when X is

$-\underset{R^{14}}{CHO}-$, $-O-$, or $-CHNH-$,

hydrogen ;
aryl, as defined above ;
Het, as defined above ;
unsubstituted or monosubstituted
$C_1$-$C_6$-alkyl, where the substituent is selected from the group consisting of hydroxyl ; $-CO_2H$ ; $-CO_2R^{11}$ or $-CONH-R^{11}$, wherein $R^{11}$ is as defined above ; $-O-COR^{12}$, wherein $R^{12}$ is as defined above ; amino ; mono- or di-$C_1$-$C_4$-alkylamino ; $-N(R^{13})-COR^{12}$ or $-CON(R^{13})-R^{11}$, where $R^{13}$, $R^{12}$ and

$R^{11}$ are as defined above; -CO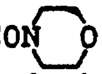; aryl, as defined above; Het, as defined above; and $-^+N(R^5)_2R^9$ $A^-$ or $-^+$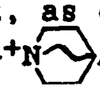$A^-$, wherein $R^5$, $R^9$

and $A^-$ are as defined above ;

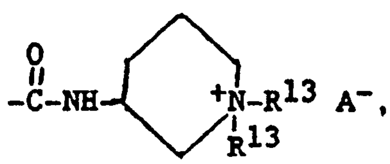

where $R^{13}$ and $A^-$ are as defined above ; or

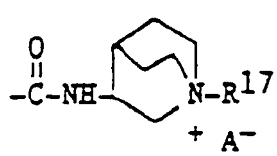

,

where $R^{17}$ is $C_1$-$C_4$-alkyl, $C_5$-$C_6$-cycloalkyl, carboxy-$C_1$-$C_4$-alkyl, carbo-$C_1$-$C_4$-alkoxy-$C_1$-$C_4$-alkyl, or $-CH_2$-aryl or $-CH_2$-Het, wherein aryl, Het and $A^-$ are as defined above ; or

$R^4$ is when X is $-CH_2-$, $-CH_2CH_2-$, $-CH=CH-$, $-CH2\underset{R15}{CH}-$, $-CH2\overset{Z}{\overset{\|}{C}}-$, or $-CH_2\underset{R^{16}}{\overset{OH}{C}}-$

hydrogen ; $C_1$-$C_7$-alkyl ; aryl, as defined above ; Het, as defined above ; $-CO_2H$ ; $-CO_2R^{11}$ or $-CONH-R^{11}$, where $R^{11}$ is as defined above ; hydroxyl ; $-O-COR^{12}$, where $R^{12}$ is as defined above ; amino ; mono- or di-$C_1$-$C_4$-alkylamino ;

$R^{12}$ and $R^{11}$ are as defined above; -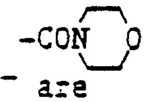; $-{}^{+}N(R^5)_2R^9$ $A^-$, where $R^5$, $R^9$ and $A^-$ are as defined above; -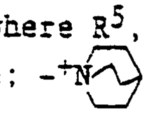 $A^-$;

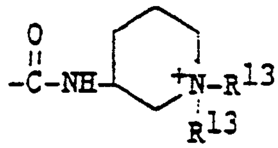 $A^-$, 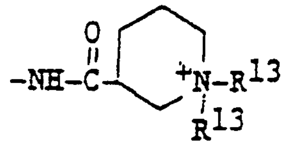 $A^-$,

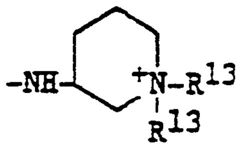 $A^-$, where $R^{13}$ and $A^-$ are as defined above;

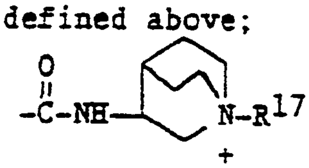 $A^-$, 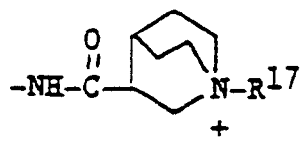 $A^-$,

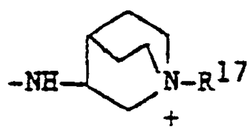 $A^-$,

where $R^{17}$ is as defined above ; substituted $C_1$-$C_5$-alkyl, wherein the substituent is selected from the group consisting of hydroxyl, $-CO_2H$, $-CO_2R^{11}$ or $-CONH-R^{11}$, where $R^{11}$ is as defined above, $-O-COR^{12}$, where $R^{12}$ is as defined above, amino, mono- or di-$C_1$-$C_4$-alkylamino, $-N(R^{13})-COR^{12}$, or

$-CON(R^{13})-R^{11}$, where $R^{13}$, $R^{12}$ and $R^{11}$ are as defined above, -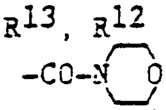, aryl, as defined above, Het, as defined above, and $-{}^{+}N(R^5)_2R^9$ $A^-$ or -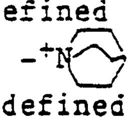 $A^-$, where $R^5$, $R^9$ and $A^-$ are as defined above,

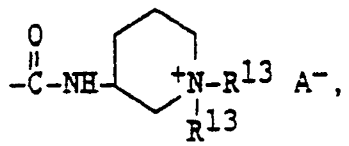 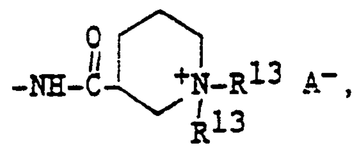

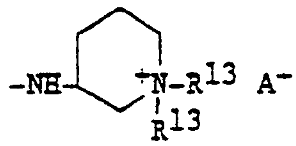 where $R^{13}$ and $A^-$ are as defined above,

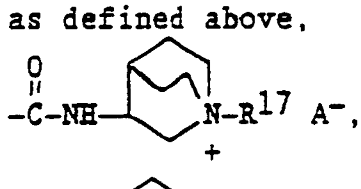 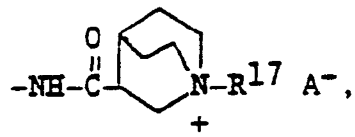

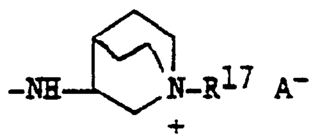 where $R^{17}$ is as defined above;

and pharmaceutically-acceptable salts thereof.

2. A peptide of Claim 1 wherein : A is $R^{10}$-CO-, $R^{11}$-O-CO-, $R^{11}$-$SO_2$-, $R^{12}$-$SO_2$, or $R^{11}$-NH-CO-, wherein $R^{10}$, $R^{11}$ and $R^{12}$ are as defined above ;
B is absent (when D is present), L-phenylalanyl or derivatives thereof substituted on the aromatic ring, or is

$$-CH_2-\underset{\displaystyle CH_2Ph}{\underset{|}{CH}}-CO- \;;$$

D is absent (when B is present), L-histidyl, N-α-methyl-L-histidyl, L-valinyl or L-nor-leucinyl ;
$R^1$ is cyclohexyl ;
$R^2$ is hydrogen or methyl ;
$R^3$ is n-propyl, 2-methylpropyl, or hydrogen ;
$R^4$ is $-CH_2-^{+}N(R^5)_2R^9\ A^-$ where $R^5$, $R^9$ and $A^-$ are defined as above, or hydrogen, when X is $-CH_2O-$, or $R^4$ is $-^{+}N(R^5)_2R^9\ A^-$ or $-CH_2-^{+}N(R^5)_2R^9\ A^-$, when X is $-CH_2CH_2-$.

3. A peptide of Claim 1 wherein the substituents for a peptide are selected from the following groups :

| A | B | D | $R^1$ | $R^2$ | X | $R^3$ | $R^4$ |
|---|---|---|---|---|---|---|---|
| Boc | Phe | His | cyclo-$C_6H_{11}$ | H | $-CH_2O-$ | H | H |
| Boc | Phe | His | cyclo-$C_6H_{11}$ | H | $-CH_2O-$ | $-CH_2CH{=}CH_2$ | H |
| Boc | Phe | His | cyclo-$C_6H_{11}$ | H | $-CH_2O-$ | $-CH_2CH_2CH_3$ | H |
| Boc | Phe | His | cyclo-$C_6H_{11}$ | H | $-CH_2O-$ | $-CH(CH_3)CH_2CH_3$ | H |
| Boc | Phe | His | cyclo-$C_6H_{11}$ | H | $-CH_2O-$ | $-CH(CH_3)CH{=}CH_2$ | H |
| Boc | Phe | His | cyclo-$C_6H_{11}$ | H | $-CH_2O-$ | $-CH_2C(CH_3){=}CH_2$ | H |
| Boc | Phe | His | cyclo-$C_6H_{11}$ | H | $-CH_2O-$ | $-CH_2CH(CH_3)_2$ | H |
| Boc | Phe | His | cyclo-$C_6H_{11}$ | H | $-CH_2O-$ | -cyclo-$C_5H_7$ | H |
| Boc | Phe | His | cyclo-$C_6H_{11}$ | H | $-CH_2O-$ | -cyclo-$C_5H_9$ | H |
| Boc | Phe | His | cyclo-$C_6H_{11}$ | H | $-CH_2O-$ | $-CH_2CH_2CH_3$ | H |
| Boc | Phe | His | cyclo-$C_6H_{11}$ | H | $-CH_2CH_2-$ | $-CH_2CH_2CH_3$ | H |
| Boc | Phe | His | cyclo-$C_6H_{11}$ | H | $-CH(CH_3)O-$ | $-CH_2CH_2CH_3$ | H |
| Boc | Phe | His | cyclo-$C_6H_{11}$ | H | $-CH_2CO-$ | $-CH_2CH_2CH_3$ | H |
| Boc | Phe | His | cyclo-$C_6H_{11}$ | H | $-CH_2CH(OH)-$ | $-CH_2CH_2CH_3$ | H |
| Boc | Phe | His | cyclo-$C_6H_{11}$ | H | $-CH_2CH-$<br>$N(CH_3)_2$ | $-CH_2CH_2CH_3$ | H |
| Boc | Phe | His | cyclo-$C_6H_{11}$ | H | $-CH_2NH-$ | $-CH_2CH_2CH_3$ | H |
| Boc | Phe | His | cyclo-$C_6H_{11}$ | $CH_3$ | $-CH_2O-$ | H | H |
| Boc | Phe | His | cyclo-$C_6H_{11}$ | $CH_3$ | $-CH_2O-$ | $-CH_2CH_2CH_3$ | H |
| Boc | Phe | His | cyclo-$C_6H_{11}$ | $CH_3$ | $-CH_2O-$ | H | $-CH_2NH_2$ |
| Boc | Phe | His | cyclo-$C_6H_{11}$ | $CH_3$ | $-CH_2CH_2-$ | $-CH_2CH_2CH_3$ | H |
| Boc | Phe | His | cyclo-$C_6H_{11}$ | $CH_3$ | $-CH_2CO-$ | $-CH_2CH_2CH_3$ | H |
| Boc | Phe | His | cyclo-$C_6H_{11}$ | $CH_3$ | $-CH_2CH(OH)-$ | $-CH_2CH_2CH_3$ | H |
| Boc | Phe | His | cyclo-$C_6H_{11}$ | $CH_3$ | $-CH_2CH-$<br>$N(CH_3)_2$ | $-CH_2CH_2CH_3$ | H |

-continued-

| A | B | D | $R^1$ | $R^2$ | X | $R^3$ | $R^4$ |
|---|---|---|---|---|---|---|---|
| Boc | Phe | His | cyclo-$C_6H_{11}$ | $CH_3$ | $-CH_2CH-$<br>$N(CH_3)_2$ | H | H |
| Boc | Phe | His | cyclo-$C_6H_{11}$ | $CH_3$ | $-CH_2CH-$<br>$NH_2$ | H | H |
| Boc | Phe | His | cyclo-$C_6H_{11}$ | $CH_3$ | $-CH_2CO-$ | H | $-CH_2N(CH_3)_2$ |
| Boc | Phe | His | cyclo-$C_6H_{11}$ | $CH_3$ | $-CH_2CH(OH)-$ | H | $-CH_2N(CH_3)_2$ |
| Boc | Phe | His | cyclo-$C_6H_{11}$ | $CH_3$ | $-CH_2CO-$ | H | $-CH_2N(CH_3)_3^+$<br>$OAc^-$ |
| Boc | Phe | His | cyclo-$C_6H_{11}$ | $CH_3$ | $-CH_2CH(OH)-$ | H | $-CH_2N(CH_3)_3^+$<br>$OAc^-$ |
| Boc | Phe | His | cyclo-$C_6H_{11}$ | H | -O- | $-CH_2CH_2CH_3$ | H |
| Boc | Phe | His | cyclo-$C_6H_{11}$ | H | -O- | -Phenyl | H |
| Boc | Phe | His | cyclo-$C_6H_{11}$ | $CH_3$ | -O- | $-CH_2CH_2CH_3$ | H |
| Boc | Phe | His | cyclo-$C_6H_{11}$ | $CH_3$ | $-CH_2CO-$ | $-(CH_2)_2N(CH_3)_2$ | H |
| Boc | Phe | His | cyclo-$C_6H_{11}$ | $CH_3$ | $-CH_2CO-$ | $-CH_2CH_2OH$ | H |
| Etoc | Phe | His | cyclo-$C_6H_{11}$ | H | $-CH_2O-$ | H | H |
| Etoc | Phe | His | cyclo-$C_6H_{11}$ | H | $-CH_2O-$ | $-CH_2CH=CH_2$ | H |
| Etoc | Phe | His | cyclo-$C_6H_{11}$ | H | $-CH_2O-$ | $-CH_2CH_2CH_3$ | H |
| Etoc | Phe | His | cyclo-$C_6H_{11}$ | H | $-CH_2O-$ | $-CH(CH_3)CH_2CH_3$ | H |
| Etoc | Phe | His | cyclo-$C_6H_{11}$ | H | $-CH_2O-$ | $-CH(CH_3)CH=CH_2$ | H |
| Etoc | Phe | His | cyclo-$C_6H_{11}$ | H | $-CH_2O-$ | $-CH_2C(CH_3)=CH_2$ | H |
| Etoc | Phe | His | cyclo-$C_6H_{11}$ | H | $-CH_2O-$ | $-CH_2CH(CH_3)_2$ | H |
| Etoc | Phe | His | cyclo-$C_6H_{11}$ | H | $-CH_2O-$ | -cyclo-$C_5H_7$ | H |
| Etoc | Phe | His | cyclo-$C_6H_{11}$ | H | $-CH_2O-$ | -cyclo-$C_5H_9$ | H |
| Etoc | Phe | His | cyclo-$C_6H_{11}$ | H | $-CH_2O-$ | $-CH_2CH_2CH_3$ | H |
| Etoc | Phe | His | cyclo-$C_6H_{11}$ | H | $-CH_2CH_2-$ | $-CH_2CH_2CH_3$ | H |
| Etoc | Phe | His | cyclo-$C_6H_{11}$ | H | $-CH(CH_3)O-$ | $-CH_2CH_2CH_3$ | H |

-continued-

| A | B | D | $R^1$ | $R^2$ | X | $R^3$ | $R^4$ |
|---|---|---|---|---|---|---|---|
| Etoc | Phe | His | cyclo-$C_6H_{11}$ | H | $-CH_2CO-$ | $-CH_2CH_2CH_3$ | H |
| Etoc | Phe | His | cyclo-$C_6H_{11}$ | H | $-CH_2CH(OH)-$ | $-CH_2CH_2CH_3$ | H |
| Etoc | Phe | His | cyclo-$C_6H_{11}$ | H | $-CH_2CH-$<br>$N(CH_3)_2$ | $-CH_2CH_2CH_3$ | H |
| Etoc | Phe | His | cyclo-$C_6H_{11}$ | H | $-CH_2CH-$<br>$NH_2$ | H | H |
| Etoc | Phe | His | cyclo-$C_6H_{11}$ | $CH_3$ | $-CH_2O-$ | H | H |
| Etoc | Phe | His | cyclo-$C_6H_{11}$ | $CH_3$ | $-CH_2O-$ | $-CH_2CH_2CH_3$ | H |
| Etoc | Phe | His | cyclo-$C_6H_{11}$ | $CH_3$ | $-CH_2O-$ | H | $-CH_2NH_2$ |
| Etoc | Phe | His | cyclo-$C_6H_{11}$ | $CH_3$ | $-CH_{32}CH_2-$ | $-CH_2CH_2CH_3$ | H |
| Etoc | Phe | His | cyclo-$C_6H_{11}$ | $CH_3$ | $-CH_2CO-$ | $-CH_2CH_2CH_3$ | H |
| Etoc | Phe | His | cyclo-$C_6H_{11}$ | $CH_3$ | $-CH_2CH(OH)-$ | $-CH_2CH_2CH_3$ | H |
| Etoc | Phe | His | cyclo-$C_6H_{11}$ | $CH_3$ | $-CH_2CH-$<br>$N(CH_3)_2$ | $-CH_2CH_2CH_3$ | H |
| Etoc | Phe | His | cyclo-$C_6H_{11}$ | $CH_3$ | $-CH_2CH-$<br>$N(CH_3)_2$ | H | H |
| Etoc | Phe | His | cyclo-$C_6H_{11}$ | $CH_3$ | $-CH_2NH-$ | H | H |
| Etoc | Phe | His | cyclo-$C_6H_{11}$ | $CH_3$ | $-CH_2CO-$ | H | $-CH_2N(CH_3)_2$ |
| Etoc | Phe | His | cyclo-$C_6H_{11}$ | $CH_3$ | $-CH_2CH(OH)-$ | H | $-CH_2N(CH_3)_2$ |
| Etoc | Phe | His | cyclo-$C_6H_{11}$ | $CH_3$ | $-CH_2CO-$ | H | $-CH_2N(CH_3)_3^+$<br>$OAc^-$ |
| Etoc | Phe | His | cyclo-$C_6H_{11}$ | $CH_3$ | $-CH_2CH(OH)-$ | H | $-CH_2N(CH_3)_3^+$<br>$OAc^-$. |

**4.** A peptide of Claim 1 which is :

| A | B | D | $R^1$ | $R^2$ | X | $R^3$ | $R^4$ |
|---|---|---|---|---|---|---|---|
| Etoc | Phe | His | cyclo-$C_6H_{11}$ | H | $-CH_2O-$ | H | $-CH_2NH_2$ |
| Etoc | Phe | His | cyclo-$C_6H_{11}$ | H | $-CH_2O-$ | H | $-CO_2H$ |
| Etoc | Phe | His | cyclo-$C_6H_{11}$ | H | $-CH_2O-$ | H | $-CONHCH_2CH_2N(CH_3)_2$ |
| Etoc | Phe | His | cyclo-$C_6H_{11}$ | H | $-CH_2O-$ | H | $-CH_2NHCO-$ 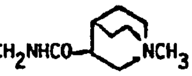 + ⁻OAc |
| Etoc | Phe | His | cyclo-$C_6H_{11}$ | H | $-CH_2O-$ | H | $-CONH-$ 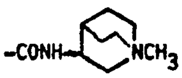 + ⁻OAc |
| Etoc | Phe | His | cyclo-$C_6H_{11}$ | $CH_3$ | $-CH_2O$ | H | $-CH_2NH_2$ |
| Etoc | Phe | His | cyclo-$C_6H_{11}$ | $CH_3$ | $-CH_2O$ | H | $-CO_2H$ |
| Etoc | Phe | His | cyclo-$C_6H_{11}$ | $CH_3$ | $-CH_2O$ | H | $-CONHCH_2CH_2N(CH_3)_2$ |
| Etoc | Phe | His | cyclo-$C_6H_{11}$ | H | $-CH_2O-$ | H | $-CH_2NHCO-$ 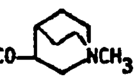 + ⁻OAc |
| Etoc | Phe | His | cyclo-$C_6H_{11}$ | H | $-CH_2O-$ | H | $-CONH-$ 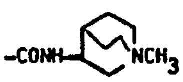 + ⁻OAc |
| Etoc | Phe | Nle | cyclo-$C_6H_{11}$ | H | $-CH_2O-$ | H | H |
| Etoc | Phe | Nle | cyclo-$C_6H_{11}$ | H | $-CH_2O-$ | H | $-CH_2NH_2$ |
| Etoc | Phe | Nle | cyclo-$C_6H_{11}$ | H | $-CH_2O-$ | H | $-CO_2H$ |
| Etoc | Phe | Nle | cyclo-$C_6H_{11}$ | H | $-CH_2O-$ | H | $-CONHCH_2CH_2N(CH_3)_2$ |
| Etoc | Phe | Nle | cyclo-$C_6H_{11}$ | H | $-CH_2O-$ | H | $-CH_2NHCO-$ 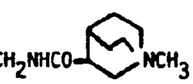 + ⁻OAc |

-continued-

| A | B | D | $R^1$ | $R^2$ | X | $R^3$ | $R^4$ |
|---|---|---|---|---|---|---|---|
| Etoc | Phe | Nle | cyclo-$C_6H_{11}$ | H | $-CH_2O-$ | H | 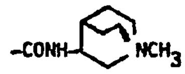 + $^{-}OAc$ |
| Etoc | Phe | Nle | cyclo-$C_6H_{11}$ | $CH_3$ | $-CH_2O-$ | H | $-CH_2NH_2$ |
| Etoc | Phe | Nle | cyclo-$C_6H_{11}$ | $CH_3$ | $-CH_2O-$ | H | $-CO_2H$ |
| Etoc | Phe | Nle | cyclo-$C_6H_{11}$ | $CH_3$ | $-CH_2O-$ | H | $-CONHCH_2CH_2N(CH_3)_2$ |
| Etoc | Phe | Nle | cyclo-$C_6H_{11}$ | $CH_3$ | $-CH_2O-$ | H | 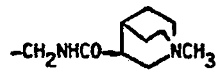 + $^{-}OAc$ |
| Etoc | Phe | Nle | cyclo-$C_6H_{11}$ | $CH_3$ | $-CH_2O-$ | H | 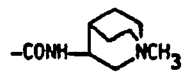 + $^{-}OAc$ |
| Etoc | Phe | His | cyclo-$C_6H_{11}$ | H | $-CH_2O-$ | $-CH_2CH_2CH_3$ | $-CH_2NH_2$ |
| Etoc | Phe | His | cyclo-$C_6H_{11}$ | H | $-CH_2O-$ | $-CH_2CH_2CH_3$ | $-CO_2H$ |
| Etoc | Phe | His | cyclo-$C_6H_{11}$ | H | $-CH_2O-$ | $-CH_2CH_2CH_3$ | $CONHCH_2CH_2N(CH_3)_2$ |
| Etoc | Phe | His | cyclo-$C_6H_{11}$ | H | $-CH_2O-$ | $-CH_2CH_2CH_3$ | 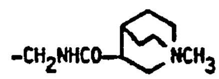 + $^{-}OAc$ |
| Etoc | Phe | His | cyclo-$C_6H_{11}$ | H | $-CH_2O-$ | $-CH_2CH_2CH_3$ | 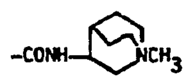 + $^{-}OAc$ |
| Etoc | Phe | His | cyclo-$C_6H_{11}$ | H | $-CH_2O-$ | $-CH_2CH_2CH_3$ | $-CH_2NH_2$ |
| Etoc | Phe | His | cyclo-$C_6H_{11}$ | H | $-CH_2O-$ | $-CH_2CH_2CH_3$ | $-CO_2H$ |
| Etoc | Phe | His | cyclo-$C_6H_{11}$ | H | $-CH_2O-$ | $-CH_2CH_2CH_3$ | $-CONHCH_2CH_2N(CH_3)_2$ |

-continued-

| A | B | D | $R^1$ | $R^2X$ | $R^3$ | R4 |
|---|---|---|---|---|---|---|
| Etoc | Phe | His | cyclo-$C_6H_{11}$ | H · $-CH_2O-$ | $-CH_2CH_2CH_3$ | 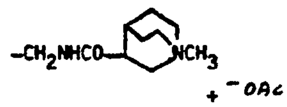 |
| Etoc | Phe | His | cyclo-$C_6H_{11}$ | H $-CH_2O-$ | $-CH_2CH_2CH_3$ | 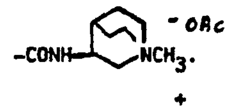 |

5. A peptide of Claim 1 wherein the substituents for a peptide are selected from the following groups :

| A | B | D | $R^1$ | $R^2$ | X | $R^3$ | $R^4$ |
|---|---|---|---|---|---|---|---|
| 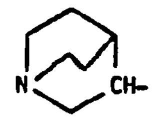 | Phe | His | cyclo-$C_6H_{11}$ | H | $-CH_2O-$ | $-CH_2CH_2CH_3$ | H |
| 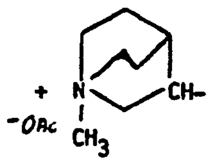 | Phe | His | cyclo-$C_6H_{11}$ | H | $-CH_2O-$ | $-CH_2CH_2CH_3$ | H |
| 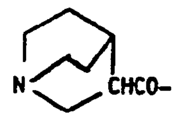 | Phe | His | cyclo-$C_6H_{11}$ | H | $-CH_2O-$ | $-CH_2CH_2CH_3$ | H |
| 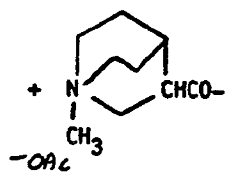 | Phe | His | cyclo-$C_6H_{11}$ | H | $-CH_2O-$ | $-CH_2CH_2CH_3$ | H |
| 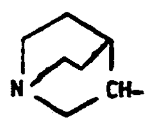 | Phe | Nle | cyclo-$C_6H_{11}$ | H | $-CH_2O-$ | $-CH_2CH_2CH_3$ | H |
| 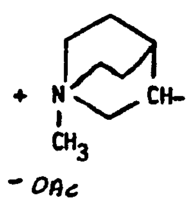 | Phe | Nle | cyclo-$C_6H_{11}$ | H | $-CH_2O-$ | $-CH_2CH_2CH_3$ | H |

-continued-

| A | B | D | $R^1$ | $R^2$ | X | $R^3$ | $R^4$ |
|---|---|---|---|---|---|---|---|
| 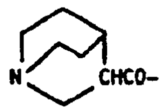 | Phe | Nle | cyclo-$C_6H_{11}$ | H | $-CH_2O-$ | $-CH_2CH_2CH_3$ | H |
| 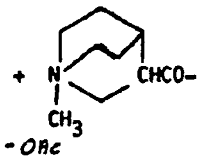 | Phe | Nle | cyclo-$C_6H_{11}$ | H | $-CH_2O-$ | $-CH_2CH_2CH_3$ | H |
| 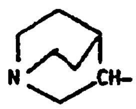 | Phe | His | cyclo-$C_6H_{11}$ | H | $CH_2CH_2$ | $-CH_2CH_2CH_3$ | H |
| 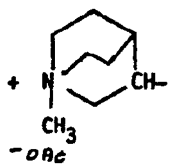 | Phe | His | cyclo-$C_6H_{11}$ | H | $CH_2CH_2$ | $-CH_2CH_2CH_3$ | H |
| 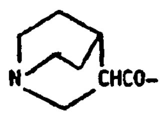 | Phe | His | cyclo-$C_6H_{11}$ | H | $CH_2CH_2$ | $-CH_2CH_2CH_3$ | H |
| 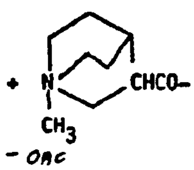 | Phe | His | cyclo-$C_6H_{11}$ | H | $CH_2CH_2$ | $-CH_2CH_2CH_3$ | H |
| 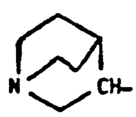 | Phe | Nle | cyclo-$C_6H_{11}$ | H | $CH_2CH_2$ | $-CH_2CH_2CH_3$ | H |

-continued-

| A | B | D | $R^1$ | $R^2$ | X | $R^3$ | $R^4$ |
|---|---|---|---|---|---|---|---|
| 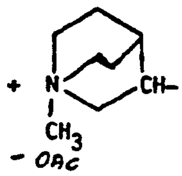 | Phe | Nle | cyclo-$C_6H_{11}$ | H | $CH_2CH_2$ | $-CH_2CH_2CH_3$ | H |
| 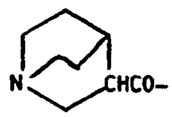 | Phe | Nle | cyclo-$C_6H_{11}$ | H | $CH_2CH_2$ | $-CH_2CH_2CH_3$ | H |
| 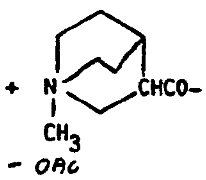 | Phe | Nle | cyclo-$C_6H_{11}$ | H | $CH_2CH_2$ | $-CH_2CH_2CH_3$ | H |
| 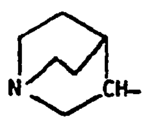 | Phe | His | cyclo-$C_6H_{11}$ | H | $CH_2CH(OH)$ | $-CH_2CH_2CH_3$ | H |
| 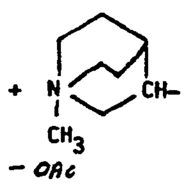 | Phe | His | cyclo-$C_6H_{11}$ | H | $CH_2CH(OH)$ | $-CH_2CH_2CH_3$ | H |
| 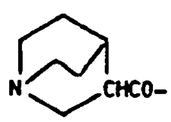 | Phe | His | cyclo-$C_6H_{11}$ | H | $CH_2CH(OH)$ | $-CH_2CH_2CH_3$ | H |
| 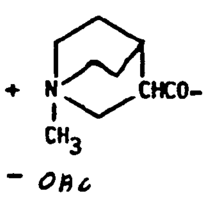 | Phe | His | cyclo-$C_6H_{11}$ | H | $CH_2CH(OH)$ | $-CH_2CH_2CH_3$ | H |

-continued-

| A | B | D | $R^1$ | $R^2$ | X | $R^3$ | $R^4$ |
|---|---|---|---|---|---|---|---|
| 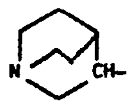 | Phe | Nle | cyclo-$C_6H_{11}$ | H | $CH_2CH(OH)$ | $-CH_2CH_2CH_3$ | H |
| 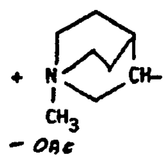 | Phe | Nle | cyclo-$C_6H_{11}$ | H | $CH_2CH(OH)$ | $-CH_2CH_2CH_3$ | H |
| 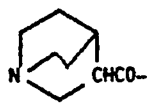 | Phe | Nle | cyclo-$C_6H_{11}$ | H | $CH_2CH(OH)$ | $-CH_2CH_2CH_3$ | H |
| 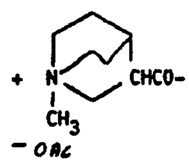 | Phe | Nle | cyclo-$C_6H_{11}$ | H | $CH_2CH(OH)$ | $-CH_2CH_2CH_3$ | H. |

6. A peptide of Claim 1 which is :
Boc-Phe-His-(ACHP)-5(S)-allyl-7-oxa-9(S)-indolizidin-3-one-2(S)-yl ;
Boc-Phe-His-(ACHP)-5(S)-(2-methallyl)-7-oxa-9(S)-indolizidin-3-one-2(S)-yl ;
Boc-Phe-His-(ACHP)-5(S)-(2-methylpropyl)-7-oxa-9(S)-indolizidin-3-one-2(S)-yl ;
Boc-Phe-His-(ACHP)-5(S)-cyclopentyl-7-oxa-9(S)-indolizidin-3-one-2(S)-yl ;
Boc-Phe-His-(ACHP)-5(S)-propyl-7-oxa-9(S)-indolizidin-3-one-2(S)-yl ; or
Boc-Phe-His-(ACHP)-6(S)-methyl-9(S)-indolizidin-3-one-2(S)-yl.

7. A pharmaceutical composition for renin-associated hypertension or congestive heart failure comprising a pharmaceutical carrier and a therapeutically-effective amount of a peptide according to Claim 1.

8. The use of a peptide according to Claim 1 for the manufacture of a medicament suitable for treating renin-associated hypertension or congestive heart failure in mammals.

9. The use of the renin inhibitor of Claim 1 for the manufacture of a medicament for treating AIDS.

10. The use of the renin inhibitor of Claim 1 for the manufacture of a medicament for treating infection by HIV.